# EUROPEAN PATENT SPECIFICATION

(11) **EP 2 924 439 B1**
(45) Date of publication and mention of the grant of the patent: **01.02.2017**
(21) Application number: 15157659.2
(22) Date of filing: 25.03.2011
(51) Int. Cl.: G01N 33/68

(54) **LTBP2 AS A BIOMARKER FOR PREDICTING OR PROGNOSTICATING MORTALITY**
LTBP2 ALS BIOMARKER ZUR VORHERSAGE ODER PROGNOSE VON MORTALITÄT
LTBP2 COMME BIOMARQUEUR POUR LA PREDICTION OU LE PRONOSTIC DE LA MORTALITE

(30) Priority: 26.03.2010 US 318064 P; 26.03.2010 EP 10158061
(43) Date of publication of application: 30.09.2015
(62) Divisional of application: 11714716.5
(73) Proprietor: MyCartis N.V., 9052 Gent (BE)
(72) Inventor: Kas, Koen, 2970 Schilde (BE)
(74) Representative: Cabinet Laurent & Charras

(56) References cited:
- WO-A1-2008/046509
- WO-A2-2004/075835
- WO-A2-2010/022210

## Description

The invention relates to protein- and/or peptide-based biomarkers useful for predicting, diagnosing, prognosticating and/or monitoring diseases and conditions in subjects, and to related methods, kits and devices.

### BACKGROUND OF THE INVENTION

In many diseases and conditions, a favourable outcome of prophylactic and/or therapeutic treatments is strongly correlated with early and/or accurate prediction, diagnosis, prognosis and/or monitoring of a disease or condition. Therefore, there exists a continuous need for additional and preferably improved manners for early and/or accurate prediction, diagnosis, prognosis and/or monitoring of diseases and conditions to guide the treatment choices.

The mammalian renal system plays central roles *inter alia* in the removal of catabolic waste products from the bloodstream and in the maintenance of fluid and electrolyte balances in the body.

Renal dysfunction encompasses diseases and conditions in which kidney function is inadequate, such as for example diseases and conditions characterised by an acute or chronic deterioration of kidney function, more particularly characterised by an acute or chronic decline in kidney excretory function, as evidenced for example by reduced glomerular filtration rate. Renal dysfunction may develop into a life-threatening condition in which the (systemic) build-up of catabolic waste products and other harmful or toxic substances and/or the development of significant imbalances in bodily fluids or electrolytes may lead to, contribute to or exacerbate the failure of other major organ systems and death.

Signs and symptoms of renal dysfunction may include *inter alia* increased levels of urea in the blood, volume overload and swelling, abnormal acid levels, abnormal levels of potassium, calcium and/or phosphate, changes in urination, fatigue, skin rash or itching, nausea, dyspnea, reduced kidney size, haematuria and anaemia. However, renal dysfunction is frequently insidious and may progress to an advanced stage before the patient notices problems and decides to seek a physician. Therefore, renal dysfunction is commonly diagnosed late, and the patient may already be in need of radical and non-trivial treatments such as dialysis or kidney transplantation.

To aid diagnosis of renal dysfunction, some methods have been developed previously. For example, one way is to determine the glomerular filtration rate (GFR). However, GFR measurements rely on invasive, time-consuming and expensive procedures involving the injection of exogenous and potentially harmful diagnostic substances and measuring their excretion at specified time period(s). Another method is to measure serum creatinine clearance. Creatinine originates from muscle tissue and is increasingly secreted by renal tubules concomitant with decreasing renal function. However, serum creatinine levels depend on age, sex, diet, muscle mass, ethnic background, physical activity, disease, other manners of secretion, *etc.,* which factors may impair the reliability of creatinine clearance for diagnosis of renal dysfunction. A further endogenous biomarker for diagnosing renal dysfunction is Cystatin C. Advantageously, compared to creatinine the expression of Cystatin C is comparably steady. Nevertheless, Cystatin C does show some limitations: for example, its levels are affected by immunosuppressive therapeutics and are dependent on thyroid function. Cystatin C also does not react rapidly enough to acute changes in GFR and is thus not a satisfactory marker for acute kidney injury (AKI). Another endogenous marker is neutrophil gelatinase-associated lipocalin (NGAL) which appears to detect early stages of acute renal injury. However, the use of NGAL is confounded by its anti-inflammatory role, which may lead to substandard specificity in complicated patient populations.

Dependable and preferably early detection and intervention is critical to effective treatment of renal dysfunction. Consequently, provision of further, alternative and preferably improved markers and tools for diagnosis, prediction, prognosis and/or monitoring of renal dysfunction continues to be of prime importance.

WO 2004/075835 speculates that LTBP2 is associated with cardiovascular diseases, hematological diseases, neurological diseases, respiratory diseases, gastroenterological diseases, and urological diseases. However, the expression profile of LTBP2 was only studied in tissue samples. Tissue samples are not a convenient source of biological sample for diagnosis purposes.WO 2008/046509 demonstrates on mRNA level the regulated expression of LTBP2 in some tissues and speculates the use of LTBP2 as a marker for *inter alia* cardiovascular diseases. Tissue samples are not a convenient source of biological sample for diagnosis purposes.

The present invention addresses the above needs in the art by identifying biomarkers for renal dysfunction and related diseases and conditions and providing uses therefore.

### SUMMARY OF THE INVENTION

Having conducted extensive experiments and tests, the inventors have found that levels of latent transforming growth factor beta binding protein 2 (LTBP2) are closely indicative of kidney function. In particular, in clinical samples from 299 patients LTBP2 showed a significant association with several tested clinical parameters related to kidney function, among others estimated glomerular filtration rate (eGFR), creatinine levels, blood urea nitrogen (BU N) levels, history of kidney failure and Cystatin C levels.

Further, for discriminating subjects with decreased GFR (< 60 ml/min/1.73m²) from subjects with normal GFR, the median AUC value (area under the ROC curve; "ROC" stands for receiver operating characteristic) is at least comparable between LTBP2 (0.9) and Cystatin C (0.92). The AUC value is a combined measure of sensitivity and specificity and a higher AUC value approaching 1) in general indicates an improved performance of the test.

Accordingly, the inventors have realised LTBP2 as a new biomarker advantageous for evaluating renal function.

Further disclosed is a method for determining renal function in a subject comprising measuring the quantity of LTBP2 in a sample from said subject. Particularly disclosed is a method for predicting, diagnosing, prognosticating and/or monitoring renal dysfunction in a subject comprising measuring LTBP2 levels in a sample from said subject. As used throughout this specification, measuring the levels of LTBP2 and/or other biomarker(s) in a sample from a subject may particularly denote that the examination phase of a method comprises measuring the quantity of LTBP2 and/or other biomarker(s) in the sample from the subject. One understands that methods of prediction, diagnosis, prognosis and/or monitoring of diseases and conditions generally comprise an examination phase in which data is collected from and/or about the subject.

In an embodiment, a method for predicting, diagnosing and/or prognosticating renal dysfunction comprises the steps of: (i) measuring the quantity of LTBP2 in a sample from the subject; (ii) comparing the quantity of LTBP2 measured in (i) with a reference value of the quantity of LTBP2, said reference value representing a known prediction, diagnosis and/or prognosis of renal dysfunction or normal renal function; (iii) finding a deviation or no deviation of the quantity of LTBP2 measured in (i) from the reference value; and (iv) attributing said finding of deviation or no deviation to a particular prediction, diagnosis and/or prognosis of renal dysfunction or normal renal function in the subject.

The method for predicting, diagnosing and/or prognosticating renal dysfunction, and in particular such method comprising steps (i) to (iv) as set forth in the previous paragraph, may be performed for a subject at two or more successive time points and the respective outcomes at said successive time points may be compared, whereby the presence or absence of a change between the prediction, diagnosis and/or prognosis of renal dysfunction at said successive time points is determined. The method thus allows to monitor a change in the prediction, diagnosis and/or prognosis of renal dysfunction in a subject over time.

In an embodiment, a method for monitoring renal dysfunction comprises the steps of: (i) measuring the quantity of LTBP2 in samples from a subject from two or more successive time points; (ii) comparing the quantity of LTBP2 between the samples as measured in (i); (iii) finding a deviation or no deviation of the quantity of LTBP2 between the samples as compared in (ii); and (iv) attributing said finding of deviation or no deviation to a change in renal function or renal dysfunction in the subject between the two or more successive time points. The method thus allows to monitor renal dysfunction or renal function in a subject over time.

Throughout the present disclosure, methods suitable for monitoring any one condition or disease as taught herein can *inter alia* allow to predict the occurrence of the condition or disease, or to monitor the progression, aggravation, alleviation or recurrence of the condition or disease, or response to treatment or to other external or internal factors, situations or stressors, *etc.* Advantageously, monitoring methods as taught herein may be applied in the course of a medical treatment of the subject, preferably medical treatment aimed at alleviating the so-monitored condition or disease. Such monitoring may be comprised, *e.g.,* in decision making whether a patient may be discharged, needs a change in treatment or needs further hospitalisation.

Similarly, throughout the present disclosure, methods suitable for prognosticating any one condition or disease as taught herein can *inter alia* allow to prognosticate the occurrence of the condition or disease, or to prognosticate the progression, aggravation, alleviation or recurrence of the condition or disease, or response to treatment or to other external or internal factors, situations or stressors, etc. may allow to prognosticate

As shown in the experimental section, clinical parameters typifying kidney dysfunction, such as for example reduced eGFR and elevated Cystatin C levels, associate with elevated levels of LTBP2. Consequently, prediction or diagnosis of renal dysfunction or a poor prognosis of renal dysfunction can in particular be associated with an elevated level of LTBP2.

For example but without limitation, an elevated quantity (i.e., a deviation) of LTBP2 in a sample from a subject compared to a reference value representing the prediction or diagnosis of no renal dysfunction (i.e., normal renal function) or representing a good prognosis for renal dysfunction respectively indicates that the subject has or is at risk of having renal dysfunction or indicates a poor prognosis for renal dysfunction in the subject (such as, e.g., a prognosis that a chronic renal dysfunction patient will progress towards end-stage kidney disease).

Renal dysfunction may be characterised by reduced GFR or eGFR. (Estimated) glomerular filtration rate may be said to be reduced compared to normal, if the GFR or eGFR is below normal by any extent. For example but without limitation: normal GFR or eGFR indicative of normal kidney function may denote values greater than 90 ml/min/1.73m²; intermediate GFR or eGFR indicative of slightly impaired kidney function may denote values between 60 and 90 ml/min/1.73m²; and reduced GFR or eGFR indicative of seriously impaired kidney function may denote values lower than 60 ml/min/1.73m².

In an exemplary but non-limiting experiment LTBP2 levels provided satisfactory discrimination between normal and reduced GFR when the threshold between normal and reduced GFR was set at 60 ml/min/1.73m². Hence, in embodiments a threshold for normal vs. reduced GFR or eGFR may be set at a value between about 50 and about 70 ml/min/1.73m², e.g., between about 55 and about 65 ml/min/1.73m², e.g., at 55, 56, 57, 58, 59, 60, 61, 62, 63, 64 or 65 ml/min/1.73m², and preferably at 60 ml/min/1.73m² wherein a value above said threshold reflects normal GFR or eGFR and a value below said threshold denotes reduced GFR or eGFR.

In other embodiments a threshold for normal vs. reduced GFR or eGFR may be set at a value between about 80 and about 100 ml/min/1.73m², e.g., between about 85 and about 95 ml/min/1.73m², e.g., at 85, 86, 87, 88, 89, 90, 91, 92, 93, 94 or 95 ml/min/1.73m², and preferably at 90 ml/min/1.73m² wherein a value above said threshold reflects normal GFR or eGFR and a value below said threshold denotes reduced GFR or eGFR.

In an exemplary but non-limiting experiment LTBP2 levels provided satisfactory discrimination between normal, intermediate and reduced GFR when the threshold between normal and intermediate GFR was set at 90 ml/min/1.73m² and the threshold between intermediate and reduced GFR was set at 60 ml/min/1.73m²

Hence, in yet other embodiments, a threshold for intermediate vs. reduced GFR or eGFR may be set at a value between about 50 and about 70 ml/min/1.73m², e.g., between about 55 and about 65 ml/min/1.73m², e.g., at 55, 56, 57, 58, 59, 60, 61, 62, 63, 64 or 65 ml/min/1.73m², and preferably at 60 ml/min/1.73m² wherein a value above said threshold reflects intermediate GFR or eGFR and a value below said threshold denotes reduced GFR or eGFR; and a further threshold for normal vs. intermediate GFR or eGFR may be set at a value between about 80 and about 100 ml/min/1.73m², e.g., between about 85 and about 95 ml/min/1.73m², e.g., at 85, 86, 87, 88, 89, 90, 91, 92, 93, 94 or 95 ml/min/1.73m², and preferably at 90 ml/min/1.73m² wherein a value above said threshold reflects normal GFR or eGFR and a value below said threshold denotes intermediate GFR or eGFR.

As taught herein, the level of LTBP2, such as for example the LTBP2 concentration in plasma and /or urine, correlates with glomerular filtration rate (GFR). Consequently, the quantity of LTBP2 as measured in a subject can be converted to a GFR value in order to determine or estimate the latter. A suitable conversion formula for such purpose may also include additional factors such as clinical parameters (without limitation, height, age, sex, race, muscle mass, *etc.)* and/or clinical variables (e.g., blood-measured variables such as without limitation hematocrite, albumin concentration, thyroid hormones, *etc.).* Consequently, it is herein disclosed a method for determining glomerular filtration rate (GFR) of a subject comprising measuring the quantity of LTBP2 in a sample from said subject and converting said measured quantity of LTBP2 to GFR of said subject.

The quantity of LTBP2 as measured in a subject may be converted to a GFR value as a part or step of the herein disclosed diagnosis, prediction, prognosis and/or monitoring methods. So-calculated GFR values may be compared with known GFR values representing various stages of GFR and kidney function impairment. The quantity of LTBP2 may thus be used to determine the degree of GFR reduction in a subject.

Accordingly, in an embodiment of the herein disclosed diagnosis, prediction, prognosis and/or monitoring methods the renal dysfunction may encompass, denote or correspond to GFR reduction.

Also disclosed is a method to determine whether a subject is or is not (such as, for example, still is, or is no longer) in need of a therapy to treat renal dysfunction, comprising: (i) measuring the quantity of LTBP2 in the sample from the subject; (ii) comparing the quantity of LTBP2 measured in (i) with a reference value of the quantity of LTBP2, said reference value representing a known diagnosis, prediction and/or prognosis of renal dysfunction or normal renal function; (iii) finding a deviation or no deviation of the quantity of LTBP2 measured in (i) from said reference value; (iv) inferring from said finding the presence or absence of a need for a therapy to treat renal dysfunction. A therapy may be particularly indicated where steps (i) to (iii) allow for a conclusion that the subject has or is at risk of having renal dysfunction or has a poor prognosis for renal dysfunction, such as for example but without limitation, where the quantity of LTBP2 in the sample from the subject is elevated (i.e., a deviation) compared to a reference value representing the prediction or diagnosis of no renal dysfunction (i.e., normal renal function). Without limitation, a patient having renal dysfunction upon admission to or during stay in a medical care centre may be tested as taught herein for the necessity of continuing a treatment of said renal dysfunction, and may be discharged when such treatment is no longer needed or is needed only to a given limited extent.

Exemplary therapies for renal dysfunction encompass without limitation low-potassium and/or low phosphorus diets, phosphorus-lowering medications (e.g., calcium carbonate, calcitriol, sevelamer), red blood cell production stimulating agents (e.g., erythropoietin, darbepoietin), iron supplements, blood pressure medications, vitamin supplements, haemodialysis and kidney transplantation.

In embodiments, renal dysfunction as used herein may refer to acute renal failure (acute kidney injury). In other embodiments, renal dysfunction as used herein may refer to chronic renal failure (chronic kidney disease). In further embodiments, renal dysfunction as used herein may be associated or caused by fibrosis of the kidney tissue (renal fibrosis), particularly but without limitation in chronic kidney disease patients or heart failure patients.

Particularly advantageously, renal dysfunction as intended herein may involve acute renal dysfunction or AKI. As demonstrated by the inventors, LTBP2 can detect abrupt changes in renal function. Since AKI commonly entails sudden drops in GFR, the measurement of LTBP2 - as a marker rapidly reacting to such abrupt GFR changes - may be particularly suitable for diagnosing, predicting, prognosticating and/or monitoring AKI.

Using LTBP2 as a marker for AKI may be particularly useful in patients known or expected to be at risk of developing AKI. Without limitation, such LTBP2 testing or screening may be effected in the general population of intensive care unit (ICU) patients (i.e., testing a subject at ICU), such as, e.g., in patients having undergone surgery and more particularly cardiac surgery, in whom the incidence of acute kidney injury can be as high as 30-50%. Also without limitation, LTBP2 testing or screening may be employed in patients undergoing or having undergone coronary or peripheral angiography, in whom the incidence of developing contrast fluid-induced nephropathy may be as high as 5-10%. By means of example, in such situations LTBP2 may be used as a diagnostic marker (e.g., LTBP2 may be measured within a given time, e.g., within 24 hours, following the procedure) or as a predictive marker to identify patients sensitive or prone to AKI development.

As demonstrated in the examples, LTBP2 can identify subjects having renal dysfunction in a subject population presenting with (acute) dyspnea. Dyspnea (dyspnea or shortness of breath) is a common and distressing symptom which may be connected to a range of underlying pathologies, such as, e.g., lung cancer, chronic obstructive pulmonary disease (COPD), congestive or acute heart failure, and renal dysfunction. To treat a patient manifesting with dyspnea adequately, the underlying problem needs to be established.

Accordingly, in methods of diagnosing, predicting, prognosticating and/or monitoring renal dysfunction as taught herein, the subject may present with (manifest with) dyspnea. Preferably, the dyspnea may be acute dyspnea. Said methods may particularly allow to discriminate between (subjects having) dyspnea associated with or caused by renal dysfunction and (subjects having) dyspnea associated with or caused by other conditions (such as without limitation COPD or pneumonia).

As stated in the examples, the correlations between LTBP2 levels and Cystatin C levels or eGFR persist even following a correction for the presence of acute decompensated heart failure (AHF) in the subject population. Hence, LTBP2 can detect abrupt changes in renal function (eGFR) due to acute decompensation of the heart (i.e., reduced cardiac output).

Accordingly, in methods of diagnosing, predicting, prognosticating and/or monitoring renal dysfunction as taught herein, the subject may have or may be at risk of having heart failure, preferably acute decompensated heart failure (AHF). Such methods may *inter alia* allow to diagnose acute worsening of renal function associated with or caused by reduced cardiac output, or monitor renal function in the course of treatment of AHF.

As also shown in the examples, the inventors have found that LTBP2 levels upon admission in subjects manifesting with acute dyspnea were significantly higher in those subjects who will have died within one year post-admission compared to those subjects ,who will have remained alive at one year. This distinction was also observed when the patient population was divided based on the presence or absence of acute heart failure (AHF), or based on renal (dys)function as measured by GFR. Consequently, the inventors have realised LTBP2 as a new biomarker advantageous for predicting or prognosticating mortality in patients with dyspnea, particularly acute dyspnea, in patients with AHF and/or in patients with renal dysfunction, particularly chronic renal dysfunction.

Hence, provided is also a method for predicting or prognosticating mortality in a subject having dyspnea and/or acute heart failure, comprising measuring the quantity of LTBP2 in a sample from said subject. Preferably, the dyspnea may be acute dyspnea.
. Without limitation, the dyspnea may be associated with or caused by AHF; or the dyspnea may be associated with our caused by conditions other than AHF; or the subject may have AHF without dyspnea symptoms.

In an embodiment, the method for predicting or prognosticating mortality in a subject having dyspnea and/or acute heart failure comprises the steps of: (i) measuring the quantity of LTBP2 in a sample from the subject; (ii) comparing the quantity of LTBP2 measured in (i) with a reference value of the quantity of LTBP2, said reference value representing a known prediction or prognosis of mortality; (iii) finding a deviation or no deviation of the quantity of LTBP2 measured in (i) from the reference value; and (iv) attributing said finding of deviation or no deviation to a particular prediction or prognosis of mortality in the subject.

The present methods for predicting or prognosticating mortality may be preferably performed for a subject once the subject presents with or is diagnosed with dyspnea and/or acute heart failure, more preferably upon the initial (first) presentation or diagnosis of said diseases and conditions.

As shown in the experimental section, increased mortality rate in populations of dyspneic and/or AHF subjects is associated with elevated levels of LTBP2. Consequently, prediction or prognostication of increased mortality (increased risk or chance of death within a predetermined time interval) can in particular be associated with an elevated level of LTBP2.

For example but without limitation, an elevated quantity (i.e., a deviation) of LTBP2 in a sample from a subject compared to a reference value representing the prediction prognosis of a given mortality (i.e., a given, such as a normal, risk or chance of death within a predetermined time interval) indicates that the subject has a comparably greater risk of deceasing within said time interval.

Without limitation, mortality may be suitably expressed as the chance of a subject to decease within an interval of for example several months or several years from the time of performing a prediction or prognostication method, e.g., within about 6 months or within about 1 year or within about 2, about 3, about 4, about 5, about 6, about 7, about 8, about 9 or about 10 years from the time of performing the prediction or prognostication method.

In an exemplary but non-limiting experiment LTBP2 levels provided satisfactory discrimination between normal and increased mortality in dyspnea and in AHF subjects when the time interval for considering the alive vs. dead status was
set at 1 year from the time of performing the prediction or prognostication method. Hence, in embodiments mortality may be suitably expressed as the chance of a subject to decease within an interval of between 6 months and 2 years and preferably within 1 year from performing the prediction or prognostication method.

It shall be appreciated that finding of increased chance of death in a subject can guide therapeutic decisions to treat the subject's diseases or conditions.

The inventors have further found that levels of LTBP2 protein are increased in hypertrophied left ventricles of thoracic aortic constriction (TAC) animals compared to controls. Accordingly, the inventors have realised LTBP2 as a new biomarker advantageous for evaluating left ventricular hypertrophy and cardiac fibrosis. WO 2008/046509 studies the expression of LTBP2 on mRNA level in the DOCA rat model of left ventricular hypertrophy, without a conclusive result.

As herein disclosed LTBP2 is a new biomarker advantageous for evaluating preeclampsia (PE). As herein disclosed LTBP2 is a new biomarker advantageous for evaluating pregnancy-associated proteinuria (PAP).

Hence, disclosed are methods for predicting, diagnosing, prognosticating and/or monitoring any one of left ventricular hypertrophy (LVH), cardiac fibrosis (CF), PE or PAP in a subject comprising measuring LTBP2 levels in a sample from said subject.

In an embodiment, a method for predicting, diagnosing and/or prognosticating any one of LVH, CF, PE or PAP comprises the steps of: (i) measuring the quantity of LTBP2 in a sample from the subject; (ii) comparing the quantity of LTBP2 measured in (i) with a reference value of the quantity of LTBP2, said reference value representing a known prediction, diagnosis and/or prognosis of LVH, CF, PE or PAP; (iii) finding a deviation or no deviation of the quantity of LTBP2 measured in (i) from the reference value; and (iv) attributing said finding of deviation or no deviation to a particular prediction, diagnosis and/or prognosis of LVH, CF, PE or PAP in the subject.

The method for predicting, diagnosing and/or prognosticating any one of LVH, CF, PE or PAP, and in particular such method comprising steps (i) to (iv) as set forth in the previous paragraph, may be performed for a subject at two or more successive time points and the respective outcomes at said successive time points may be compared, whereby the presence or absence of a change between the prediction, diagnosis and/or prognosis of LVH, CF, PE or PAP at said successive time points is determined. The method thus allows to monitor a change in the prediction, diagnosis and/or prognosis of any one of LVH, CF, PE or PAP in a subject over time.

In an embodiment, a method for monitoring any one of LVH, CF, PE or PAP comprises the steps of: (i) measuring the quantity of LTBP2 in samples from a subject from two or more successive time points; (ii) comparing the quantity of LTBP2 between the samples as measured in (i); (iii) finding a deviation or no deviation of the quantity of LTBP2 between the samples as compared in (ii); and (iv) attributing said finding of deviation or no deviation to a change in LVH, CF, PE or PAP in the subject between the two or more successive time points. The method thus allows to monitor any one of LVH, CF, PE or PAP in a subject over time.

Prediction or diagnosis of any one of LVH, CF, PE or PAP or a poor prognosis of LVH, CF, PE or PAP can in particular be associated with an elevated level of LTBP2.

For example but without limitation, an elevated quantity (i.e., a deviation) of LTBP2 in a sample from a subject compared to a reference value representing the prediction or diagnosis of no LVH, CF, PE or PAP (i.e., healthy state) or representing a good prognosis for LVH, CF, PE or PAP respectively indicates that the subject has or is at risk of having LVH, CF, PE or PAP or indicates a poor prognosis for LVH, CF, PE or PAP in the subject.

Also disclosed is a method to determine whether a subject is or is not (such as, for example, still is, or is no longer) in need of a therapy to treat any one of LVH, CF, PE or PAP, comprising: (i) measuring the quantity of LTBP2 in the sample from the subject; (ii) comparing the quantity of LTBP2 measured in (i) with a reference value of the quantity of LTBP2, said reference value representing a known diagnosis, prediction and/or prognosis of LVH, CF, PE or PAP; (iii) finding a deviation or no deviation of the quantity of LTBP2 measured in (i) from said reference value; (iv) inferring from said finding the presence or absence of a need for a therapy to treat LVH, CF, PE or PAP.

A therapy may be particularly indicated where steps (i) to (iii) allow for a conclusion that the subject has or is at risk of having LVH, CF, PE or PAP or has a poor prognosis for LVH, CF, PE or PAP, such as for example but without limitation, where the quantity of LTBP2 in the sample from the subject is elevated (i.e., a deviation) compared to a reference value representing the prediction or diagnosis of no LVH, CF, PE or PAP (i.e., healthy state). Without limitation, a patient having LVH, CF, PE or PAP upon admission to or during stay in a medical care centre may be tested as taught herein for the necessity of continuing a treatment of said LVH, CF, PE or PAP, and may be discharged when such treatment is no longer needed or is needed only to a given limited extent.

Any one prediction, diagnosis, prognosis and/or monitoring method as taught herein may preferably allow for sensitivity and/or specificity (preferably, sensitivity and specificity) of at least 50%, at least 60%, at least 70% or at least 80%, *e.g.,* ≥ 85% or ≥ 90% or ≥95%, *e.g.,* between about 80% and 100% or between about 85% and 95%.

Reference throughout this specification to "diseases and/or conditions" encompasses any such diseases and conditions as disclosed herein insofar consistent with the context of such a recitation, in particular but without limitation including renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

The present methods for predicting, diagnosing, prognosticating and/or monitoring the diseases or conditions may be used in individuals who have not yet been diagnosed as having such (for example, preventative screening), or who have been diagnosed as having such, or who are suspected of having such (for example, display one or more characteristic symptoms), or who are at risk of developing such (for example, genetic predisposition; presence of one or more developmental, environmental or behavioural risk factors). The methods may also be used to detect various stages of progression or severity of the diseases or conditions. The methods may also be used to detect response of the diseases or conditions to prophylactic or therapeutic treatments or other interventions. The methods can furthermore be used to help the medical practitioner in deciding upon worsening, status-quo, partial recovery, or complete recovery of the patient from the diseases or conditions, resulting in either further treatment or observation or in discharge of the patient from medical care centre.

Any one of the herein described methods for predicting, diagnosing, prognosticating and/or monitoring the diseases or conditions may be employed for population screening (such as, *e.g.,* screening in a general population or in a population stratified based on one or more criteria, *e.g.,* age, gender, ancestry, occupation, presence or absence of risk factors of AHF, *etc*.)*.* In any one the methods, the subject may form part of a patient population showing symptoms of dyspnea.

Diabetes and hypertension represent major risk factors for developing renal dysfunction, more particularly (chronic) kidney failure. Hence, the present diagnosis, prediction, prognosis and/or monitoring methods may be preferably employed in such patients and patient populations, i.e., in subjects having or being at risk of having diabetes and/or hypertension (such as, e.g., in a screening setup).

The present methods enable the medical practitioner to monitor the disease progress by measuring the level of LTBP2 in a sample of the patient. For example, a decrease in LTBP2 level as compared to a prior LTBP2 level (e.g., at the time of the admission to ED) indicates the disease or condition in the subject is improving or has improved, while an increase of the LTBP2 level as compared to a prior LTBP2 level (e.g., at the time of the admission to ED) indicates the disease or condition in the subject has worsened or is worsening. Such worsening could possibly result in the recurrence of the disease or conditions.

In view of the present disclosure, also provided are:
- the use of LTBP2 as a marker (biomarker);
- the use of LTBP2 as a marker (biomarker) for any one disease or condition as taught herein;
- the use of LTBP2 for diagnosis, prediction, prognosis and/or monitoring;
- the use of LTBP2 for diagnosis, prediction, prognosis and/or monitoring of any one disease or condition as taught herein;
particularly wherein said condition or disease may be chosen from renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

In the present prediction, diagnosis, prognosis and/or monitoring methods the measurement of LTBP2 may also be combined with the assessment of one or more further biomarkers or clinical parameters relevant for the respective diseases and conditions.

Consequently, also disclosed herein are methods, wherein the examination phase of the methods further comprises measuring the presence or absence and/or quantity of one or more such other markers in the sample from the subject. In this respect, any known or yet unknown suitable marker could be used.

A reference throughout this specification to biomarkers "other than LTBP2" or "other biomarkers" generally encompasses such other biomarkers which are useful for predicting, diagnosing, prognosticating and/or monitoring the diseases and conditions as disclosed herein. By means of example and not limitation, biomarkers useful in evaluating renal dysfunction include creatinine (i.e., serum creatinine clearance), Cystatin C and neutrophil gelatinase-associated lipocalin (NGAL), beta-trace protein, kidney injury molecule 1 (KIM-1), interleukin-18 (IL-18). Further biomarkers useful in the present disclosure include *inter alia* B-type natriuretic peptide (BNP), pro-B-type natriuretic peptide (proBNP), amino terminal pro-B-type natriuretic peptide (NTproBNP) and C-reactive peptide, and fragments or precursors of any one thereof.

Hence, disclosed is a method for predicting, diagnosing and/or prognosticating the diseases or conditions as taught herein in a subject comprising the steps: (i) measuring the quantity of LTBP2 and the presence or absence and/or quantity of said one or more other biomarkers in the sample from the subject; (ii) using the measurements of (i) to establish a subject profile of the quantity of LTBP2 and the presence or absence and/or quantity of said one or more other biomarkers ; (iii) comparing said subject profile of (ii) to a reference profile of the quantity of LTBP2 and the presence or absence and/or quantity of said one or more other biomarkers, said reference profile representing a known prediction, diagnosis and/or prognosis of the conditions, symptoms and/or parameter values according to the invention; (iv) finding a deviation or no deviation of the subject profile of (ii) from the reference profile; (v) attributing said finding of deviation or no deviation to a particular prediction, diagnosis and/or prognosis of the respective diseases or conditions in the subject.

Applying said method at two or more successive time points allows for monitoring the desired diseases or conditions.

The present methods may employ reference values for the quantity of LTBP2, which may be established according to known procedures previously employed for other biomarkers. Such reference values may be established either within (*i.e.,* constituting a step of) or external to (*i.e.,* not constituting a step of) the methods of the present invention as defined herein. Accordingly, any one of the methods taught herein may comprise a step of establishing a reference value for the quantity of LTBP2, said reference value representing either (a) a prediction or diagnosis of the absence of the diseases or as taught herein or a good prognosis thereof, or (b) a prediction or diagnosis of the diseases or conditions as taught herein or a poor prognosis thereof.

A further aspect provides a method for establishing a reference value for the quantity of LTBP2, said reference value representing:
(a) a prediction or diagnosis of the absence of the diseases or conditions as taught herein or a good prognosis thereof, or
(b) a prediction or diagnosis of the diseases or conditions as taught herein or a poor prognosis thereof,
   comprising:
   (i) measuring the quantity of LTBP2 in:
      (i a) one or more samples from one or more subjects not having the respective diseases or conditions or not being at risk of having such or having a good prognosis for such, or
      (i b) one or more samples from one or more subjects having the respective diseases or conditions or being at risk of having such or having a poor prognosis for such, and
   (ii) storing the quantity of LTBP2
      (ii a) as measured in (i a) as the reference value representing the prediction or diagnosis of the absence of the respective diseases or conditions or representing the good prognosis therefore, or
      (ii b) as measured in (i b) as the reference value representing the prediction or diagnosis of the respective diseases or conditions or representing the poor prognosis therefore.

The present methods may otherwise employ reference profiles for the quantity of LTBP2 and the presence or absence and/or quantity of one or more other biomarkers, which may be established according to known procedures previously employed for other biomarkers. Such reference profiles may be established either within (*i.e.,* constituting a step of) or external to (*i.e.,* not constituting a step of) the present methods. Accordingly, the methods taught herein may comprise a step of establishing a reference profile for the quantity of LTBP2 and the presence or absence and/or quantity of said one or more other biomarkers, said reference profile representing either (a) a prediction or diagnosis of the absence of the diseases or conditions as taught herein or a good prognosis therefore, or (b) a prediction or diagnosis of the diseases or conditions as taught herein or a poor prognosis therefore.

A further aspect provides a method for establishing a reference profile for the quantity of LTBP2 and the presence or absence and/or quantity of one or more other biomarkers useful for predicting, diagnosing, prognosticating and/or monitoring the diseases or conditions as taught herein, said reference profile representing:
(a) a prediction or diagnosis of the absence of the respective diseases or conditions or a good prognosis therefore, or
(b) a prediction or diagnosis of the respective diseases or conditions or a poor prognosis therefore,
   comprising:
   (i) measuring the quantity of LTBP2 and the presence or absence and/or quantity of said one or more other biomarkers in:
      (i a) one or more samples from one or more subjects not having the respective diseases or conditions or not being at risk of having such or having a good prognosis for such; or
      (i b) one or more samples from one or more subjects having the respective diseases or conditions or being at risk of having such or having a poor prognosis for such;
   (ii)
      (ii a) using the measurements of (i a) to create a profile of the quantity of LTBP2 and the presence or absence and/or quantity of said one or more other biomarkers; or
      (ii b) using the measurements of (i b) to create a profile of the quantity of LTBP2 and the presence or absence and/or quantity of said one or more other biomarkers;
   (iii)
      (iii a) storing the profile of (ii a) as the reference profile representing the prediction or diagnosis of the absence of the respective diseases or conditions or representing the good prognosis therefore; or
      (iii b) storing the profile of (ii b) as the reference profile representing the prediction or diagnosis of the respective diseases conditions or representing the poor prognosis therefore.

Further provided is a method for establishing a LTBP2 base-line or reference value in a subject, comprising: (i) measuring the quantity of LTBP2 in the sample from the subject at different time points wherein the subject is not suffering from the diseases or conditions as taught herein, and (ii) calculating the range or mean value of the subject, which is the LTBP2 base-line or reference value for said subject.

Preferably, the subject as intended in any one of the present methods may be human.

The quantity of LTBP2 and/or the presence or absence and/or quantity of the one or more other biomarkers may be measured by any suitable technique such as may be known in the art. For example, the quantity of LTBP2 and/or the presence or absence and/or quantity of the one or more other biomarkers may be measured using, respectively, a binding agent capable of specifically binding to LTBP2 and/or to fragments thereof, and a binding agent capable of specifically binding to said one or more other biomarkers. For example, the binding agent may be an antibody, aptamer, photoaptamer, protein, peptide, peptidomimetic or a small molecule. For example, the quantity of LTBP2 and/or the presence or absence and/or quantity of the one or more other biomarkers may be measured using an immunoassay technology or a mass spectrometry analysis method or a chromatography method, or a combination of said methods.

Further disclosed is a kit for predicting, diagnosing, prognosticating and/or monitoring the diseases or conditions as taught herein in a subject, the kit comprising (i) means for measuring the quantity of LTBP2 in a sample from the subject, and optionally and preferably (ii) a reference value of the quantity of LTBP2 or means for establishing said reference value, wherein said reference value represents a known prediction, diagnosis and/or prognosis of the respective diseases or conditions. The kit thus allows one to: measure the quantity of LTBP2 in the sample from the subject by means (i); compare the quantity of LTBP2 measured by means (i) with the reference value of (ii) or established by means (ii); find a deviation or no deviation of the quantity of LTBP2 measured by means (i) from the reference value of (ii); and consequently attribute said finding of deviation or no deviation to a particular prediction, diagnosis and/or prognosis of the respective diseases or conditions in the subject.

A further embodiment provides a kit for predicting, diagnosing, prognosticating and/or monitoring the diseases or conditions as taught herein in a subject, the kit comprising (i) means for measuring the quantity of LTBP2 in a sample from the subject and (ii) means for measuring the presence or absence and/or quantity of one or more other biomarkers in the sample from the subject, and optionally and preferably (iii) means for establishing a subject profile of the quantity of LTBP2 and the presence or absence and/or quantity of said one or more other biomarkers, and optionally and preferably (iv) a reference profile of the quantity of LTBP2 and the presence or absence and/or quantity of said one or more other biomarkers, or means for establishing said reference profile, said reference profile representing a known prediction, diagnosis and/or prognosis of the conditions, symptoms and/or parameter values according to the invention. Such kit thus allows one to: measure the quantity of LTBP2 and the presence or absence and/or quantity of said one or more other biomarkers in the sample from the subject by respectively means (i) and (ii); establish (*e.g.,* using means included in the kit or using suitable external means) a subject profile of the quantity of LTBP2 and the presence or absence and/or quantity of said one or more other biomarkers based on said measurements; compare the subject profile with the reference profile of (iv) or established by means (iv); find a deviation or no deviation of said subject profile from said reference profile; and consequently attribute said finding of deviation or no deviation to a particular prediction, diagnosis and/or prognosis of the respective diseases or conditions in the subject.

The means for measuring the quantity of LTBP2 and/or the presence or absence and/or quantity of the one or more other biomarkers in the present kits may comprise, respectively, one or more binding agents capable of specifically binding to LTBP2 and/or to fragments thereof, and one or more binding agents capable of specifically binding to said one or more other biomarkers. For example, any one of said one or more binding agents may be an antibody, aptamer, photoaptamer, protein, peptide, peptidomimetic or a small molecule. For example, any one of said one or more binding agents may be advantageously immobilised on a solid phase or support. The means for measuring the quantity of LTBP2 and/or the presence or absence and/or quantity of the one or more other biomarkers in the present kits may employ an immunoassay technology or mass spectrometry analysis technology or chromatography technology, or a combination of said technologies.

Disclosed is thus also a kit for predicting, diagnosing, prognosticating and/or monitoring the diseases or conditions as taught herein comprising: (a) one or more binding agents capable of specifically binding to LTBP2 and/or to fragments thereof; (b) preferably, a known quantity or concentration of LTBP2 and/or a fragment thereof (*e.g.,* for use as controls, standards and/or calibrators); (c) preferably, a reference value of the quantity of LTBP2, or means for establishing said reference value. Said components under (a) and/or (c) may be suitably labelled as taught elsewhere in this specification.

Also disclosed is a kit for predicting, diagnosing and/or prognosticating the diseases or conditions as taught herein comprising: (a) one or more binding agents capable of specifically binding to LTBP2 and/or to fragments thereof; (b) one or more binding agents capable of specifically binding to one or more other biomarkers ; (c) preferably, a known quantity or concentration of LTBP2 and/or a fragment thereof and a known quantity or concentration of said one or more other biomarkers (*e.g.,* for use as controls, standards and/or calibrators); (d) preferably, a reference profile of the quantity of LTBP2 and the presence or absence and/or quantity of said one or more other biomarkers, or means for establishing said reference profiles. Said components under (a), (b) and/or (c) may be suitably labelled as taught elsewhere in this specification.

Further disclosed is the use of the kit as described herein for diagnosing, predicting, prognosticating and/or monitoring the diseases or conditions as taught herein.

Also disclosed are reagents and tools useful for measuring LTBP2 and optionally the one or more other biomarkers concerned herein.

Hence, disclosed is a protein, polypeptide or peptide array or microarray comprising (a) LTBP2 and/or a fragment thereof, preferably a known quantity or concentration of said LTBP2 and/or fragment thereof; and (b) optionally and preferably, one or more other biomarkers, preferably a known quantity or concentration of said one or more other biomarkers.

Also disclosed is a binding agent array or microarray comprising: (a) one or more binding agents capable of specifically binding to LTBP2 and/or to fragments thereof, preferably a known quantity or concentration of said binding agents; and (b) optionally and preferably, one or more binding agents capable of specifically binding to one or more other biomarkers, preferably a known quantity or concentration of said binding agents.

Also disclosed are kits as taught here above configured as portable devices, such as, for example, bed-side devices, for use at home or in clinical settings.

A related aspect thus provides a portable testing device capable of measuring the quantity of LTBP2 in a sample from a subject comprising: (i) means for obtaining a sample from the subject, (ii) means for measuring the quantity of LTBP2 in said sample, and (iii) means for visualising the quantity of LTBP2 measured in the sample.

In an embodiment, the means of parts (ii) and (iii) may be the same, thus providing a portable testing device capable of measuring the quantity of LTBP2 in a sample from a subject comprising (i) means for obtaining a sample from the subject; and (ii) means for measuring the quantity of LTBP2 in said sample and visualising the quantity of LTBP2 measured in the sample.

In an embodiment, said visualising means is capable of indicating whether the quantity of LTBP2 in the sample is above or below a certain threshold level and/or whether the quantity of LTBP2 in the sample deviates or not from a reference value of the quantity of LTBP2, said reference value representing a known prediction, diagnosis and/or prognosis of the diseases or conditions as taught herein. Hence, the portable testing device may suitably also comprise said reference value or means for establishing the reference value.

In an embodiment, the threshold level is chosen such that the quantity of LTBP2 in the sample above said threshold level indicates that the subject has or is at risk of having the respective disease or condition or indicates a poor prognosis for such in the subject, and the quantity of LTBP2 in the sample below said threshold level indicates that the subject does not have or is not at risk of having the diseases or conditions as taught herein or indicates a good prognosis for such in the subject.

In an embodiment, the portable testing device comprises a reference value representing the prediction or diagnosis of the absence of the diseases or conditions as taught herein or representing a good prognosis for such, or comprises means for establishing said reference value, and an elevated quantity of LTBP2 in the sample from the subject compared to said reference value indicates that the subject has or is at risk of having the respective disease or condition or indicates a poor prognosis for such in the subject. In another embodiment, the portable testing device comprises a reference value representing the prediction or diagnosis of the diseases or conditions as taught herein or representing a poor prognosis for such, or comprises means for establishing said reference value, and a comparable quantity of LTBP2 in the sample from the subject compared to said reference value indicates that the subject has or is at risk of having the respective disease or condition or indicates a poor prognosis for such in the subject.

In a further embodiment, the measuring (and optionally visualisation) means of the portable testing device may comprise a solid support having a proximal and distal end, comprising: - a sample application zone in the vicinity of the proximal end; - a reaction zone distal to the sample application zone; and - a detection zone distal to the reaction zone; - optionally control standards comprising LTBP2 protein or peptide fragments, whereby said support has a capillary property that directs a flow of fluid sample applied in the application zone in a direction from the proximal end to the distal end; and - optionally comprising a fluid source improving the capillary flow of a more viscous sample.

The reaction zone may comprise one or more bands of a LTBP2-specific binding molecules conjugated to a detection agent, which LTBP2 specific binding molecule conjugate is disposed on the solid support such that it can migrate with the capillary flow of fluid; and wherein the detection zone comprises one or more capture bands comprising a population of LTBP2 specific molecule immobilised on the solid support.

The reaction zone may additionally comprise one or more bands of capture LTBP2-specific binding molecules in an amount sufficient to prevent a threshold quantity of LTBP2 specific binding molecule conjugates to migrate to the detection zone. Alternatively, said device additionally comprises means for comparing the amount of captured LTBP2 specific binding molecule conjugate with a threshold value.

Other aspects relate to the realisation that LTBP2 may be a valuable target for therapeutic and/or prophylactic interventions in diseases and conditions as taught herein, in particular but without limitation including renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

Hence, also disclosed herein are any one and all of the following:
(1) an agent that is able to modulate the level and/or the activity of LTBP2 for use as a medicament, preferably for use in the treatment of any one disease or condition as taught herein;
(2) use of an agent that is able to modulate the level and/or the activity of LTBP2 for the manufacture of a medicament for the treatment of any one disease or condition as taught herein; or use of an agent that is able to modulate the level and/or the activity of LTBP2 for the treatment of any one disease or condition as taught herein;
(3) a method for treating any one disease or condition as taught herein in a subject in need of such treatment, comprising administering to said subject a therapeutically or prophylactically effective amount of an agent that is able to modulate the level and/or the activity of LTBP2;
(4) The subject matter as set forth in any one of (1) to (3) above, wherein the agent is able to reduce or increase the level and/or the activity of LTBP2, preferably to reduce the level and/or the activity of LTBP2.
(5) The subject matter as set forth in any one of (1) to (4) above, wherein said agent is able to specifically bind to LTBP2.
(6) The subject matter as set forth in any one of (1) to (5) above, wherein said agent is an antibody or a fragment or derivative thereof; a polypeptide; a peptide; a peptidomimetic; an aptamer; a photoaptamer; or a chemical substance, preferably an organic molecule, more preferably a small organic molecule.
(7) The subject matter as set forth in any one of (1) to (4) above, wherein the agent is able to reduce or inhibit the expression of LTBP2, preferably wherein said agent is an antisense agent; a ribozyme; or an agent capable of causing RNA interference.
(8) The subject matter as set forth in any one of (1) to (4) above, wherein said agent is able to reduce or inhibit the level and/or activity of LTBP2, preferably wherein said agent is a recombinant or isolated deletion construct of the LTBP2 polypeptide having a dominant negative activity over the native LTBP2.
(9) An assay to select, from a group of test agents, a candidate agent potentially useful in the treatment of any one disease or condition as taught herein, said assay comprising determining whether a tested agent can modulate, such as increase or reduce and preferably reduce, the level and/or activity of LTBP2.
(10) The assay as set forth in (9) above, further comprising use of the selected candidate agent for the preparation of a composition for administration to and monitoring the prophylactic and/or therapeutic effect thereof in a non-human animal model, preferably a non-human mammal model, of any one disease or condition as taught herein.
(11) The agent isolated by the assay as set forth in (10) above.
(12) A pharmaceutical composition or formulation comprising a prophylactically and/or therapeutically effective amount of one or more agents as set forth in any one of (1) to (8) or (10) above, or a pharmaceutically acceptable N-oxide form, addition salt, prodrug or solvate thereof, and further comprising one or more of pharmaceutically acceptable carriers.
(13) A method for producing the pharmaceutical composition or formulation as set forth in (12) above, comprising admixing said one or more agents with said one or more pharmaceutically acceptable carriers.

Said condition or disease as set forth in any one of (1) to (13) above may be particularly chosen from renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

Also contemplated is thus a method (a screening assay) for selecting an agent capable of specifically binding to LTBP2 (e.g., gene or protein) comprising: (a) providing one or more, preferably a plurality of, test LTBP2-binding agents; (b) selecting from the test LTBP2-binding agents of (a) those which bind to LTBP2; and (c) counter-selecting (i.e., removing) from the test LTBP2-binding agents selected in (b) those which bind to any one or more other, unintended or undesired, targets.

Binding between test LTBP2-binding agents and LTBP2 may be advantageously tested by contacting (i.e., combining, exposing or incubating) said LTBP2 with the test LTBP2-binding agents under conditions generally conducive for such binding. For example and without limitation, binding between test LTBP2-binding agents and the LTBP2 may be suitably tested in vitro; or may be tested in host cells or host organisms comprising the LTBP2 and exposed to or configured to express the test LTBP2-binding agents.

Without limitation, the LTPB2-binding or LTBP2-modulating agents may be capable of binding LTBP2 or modulating the activity and/or level of the LTBP2 in vitro, in a cell, in an organ and/or in an organism.

In the screening assays as set forth in any one of (9) and (10) above, modulation of the activity and/or level of the LTBP2 by test LTBP2-modulating agents may be advantageously tested by contacting (i.e., combining, exposing or incubating) said LTBP2 (e.g., gene or protein) with the test LTBP2-modulating agents under conditions generally conducive for such modulation. By means of example and not limitation, where modulation of the activity and/or level of the LTBP2 results from binding of the test LTBP2-modulating agents to the LTBP2, said conditions may be generally conducive for such binding. For example and without limitation, modulation of the activity and/or level of the LTBP2 by test LTBP2-modulating agents may be suitably tested in vitro; or may be tested in host cells or host organisms comprising the LPBT2 and exposed to or configured to express the test LTBP2-modulating agents.

As well contemplated are:
- LTBP2 for use as a medicament, preferably for use in the treatment of any one disease or condition as taught herein;
- use of LTBP2 for the manufacture of a medicament for the treatment of any one disease or condition as taught herein;
- use of LTBP2 for the treatment of any one disease or condition as taught herein;
- a method for treating any one disease or condition as taught herein in a subject in need of such treatment, comprising administering to said subject a therapeutically or prophylactically effective amount of LTBP2;
particularly wherein said condition or disease may be chosen from renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

These and further aspects and preferred embodiments are described in the following sections and in the appended claims.

### BRIEF DESCRIPTION OF THE DRAWINGS

**Figure 1** illustrates sequences of full length LTBP2 (SEQ ID NO.1). The signal peptide is indicated in small caps. Also indicated is the selected MASSterclass quantified peptide (pept221 - bold, italic, underlined / SEQ ID NO.2).
**Figure 2** illustrates correlation of LTBP2 levels with estimated glomerular filtration rate (eGFR) and Cystatin C levels in all patients.
**Figure 3** illustrates that LTBP2 shows comparable performance to Cystatin C in discriminating patients with reduced eGFR (herein < 60 ml/min/1.73m²) from patients with normal eGFR. Receiver operating characteristic curve of Cystatin C (dark grey) compared to LTBP2 (light grey). Calculated median area under the curve (AUC) and 95% confidence intervals are for Cystatin C: 0.92 (0.88-0.95) and for LTBP2: 0.9 (0.85-0.93).
**Figure 4** shows box and whisker plots for LTBP2 in patients with reduced (<60) and normal (>90) and intermediate eGFR (60-90).
**Figure 5****:** (A) Box and whisker plots for LTBP2 at presentation in dyspneic patients as a function of survival at 1 year. (B) Rates of death at 1 year as a function of LTBP2 decile in all dyspneic patients.
**Figure 6** illustrates box and whisker plots for LTBP2 levels at presentation as function of survival in dyspneic patients subdivided according to acute heart failure diagnosis (A) and kidney function (B). p-values shown are Wilcoxon rank sum p-values.
**Figure 7** shows receiver operating characteristic analysis comparing LTBP2 to cystatin C, CRP, BNP and NT-proBNP for predicting death at 1 year after presentation. Calculated median area under the curve (AUC) and 95% confidence intervals are: 0.77 (0.70-0.84) for LTBP2; 0.69 (0.62-0.77) for Cystatin C; 0.61 (0.55-0.68) for CRP; 0.72 (0.65-0.78) for BNP; 0.77 (0.70-0.83) for NTproBNP.
**Figure 8** Kaplan Meier survival plot illustrating the rates of death from presentation up 600 days of follow-up. The vertical grey line is the 1 year cut-off point. Among patients with high LTBP2 levels (above cut-off for maximal accuracy for predicting death at 1 year) a high mortality rate is observed. Log-rank p value is indicated.
**Figure 9** shows a ratio profile plot for LTBP2 expression in left ventricles from TAC animals and SHAM controls. The Y-axis shows the relative MASStermind ratio's and the X-axis the different animals. The lines represent the behaviour of a LTBP2 specific peptide and a closely related family member specific peptide (LTBP4) in TAC and control animals.
**Figure 10****:** Plan (A) and side view (B) of a test strip according to the invention.
**Figure 11****:** Plan view of a test cartridge according to the invention.
**Figure 12 A-B** shows a side view and a top view, respectively, of a reagent strip according to the invention comprising several test pads.
**Figure 13****:** Gene expression profile of the LTBP2 transcript in kidney from wild type and Glis2 mutant mice as represented on Gene Expression Omnibus (http://www.ncbi.nlm.nih.gov/geo/). Bars represent value measurements as extracted from original submitter-supplied GEO Sample records and reflect the measured level of abundance of an individual transcript across the samples that make up a dataset. Values are presented as arbitrary units.
**Figure 14****:** Expression pattern for LTBP2 protein in human kidney tissue as evidenced by immunohistochemistry. Dark grey indicates reactivity of anti-LTBP2 antibody with endogenous LTBP2 protein. Star indicates LTBP2 staining in the intima region, arrow indicates the expression in endothelial cells and arrowhead expression in smooth muscle cells.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The inventors realised LTBP2 as a valuable biomarker particularly for renal (dys)function and mortality in subjects having dyspnea and/or acute heart failure and/or renal dysfunction, and further for left ventricular hypertrophy, cardiac fibrosis, preeclampsia (PE) and pregnancy-associated proteinuria (PAP).

The term "biomarker" is widespread in the art and may broadly denote a biological molecule and/or a detectable portion thereof whose qualitative and/or quantitative evaluation in a subject is predictive or informative (*e.g.,* predictive, diagnostic and/or prognostic) with respect to one or more aspects of the subject's phenotype and/or genotype, such as, for example, with respect to the status of the subject as to a given disease or condition.

Reference herein to "disease(s) and/or condition(s) as taught herein" or a similar reference encompasses any such diseases and conditions as disclosed herein insofar consistent with the context of such a recitation, in particular but without limitation including renal dysfunction, dyspnea associated with or caused by renal failure, increased mortality of subjects having dyspnea and/or acute heart failure and/or renal dysfunction, left ventricular hypertrophy, cardiac fibrosis, PE and PAP.

Renal or kidney dysfunction, which may also be interchangeably known as renal or kidney failure or insufficiency, generally encompasses states, diseases and conditions in which the functioning of renal tissue is inadequate, particularly wherein kidney excretory function is compromised.

Signs and symptoms of renal dysfunction may include without limitation any one or more of increased levels of urea and/or nitrogen in the blood; lower than normal creatinine clearance and higher than normal creatinine levels in blood; lower than normal free water clearance; volume overload and swelling; abnormal acid levels; higher than normal levels of potassium, calcium and/or phosphate in blood; changes in urination (e.g., volume, osmolarity); microalbuminuria or macroalbuminuria; altered activity of kidney enzymes such as gamma glutamyl synthetase; fatigue; skin rash or itching; nausea; dyspnea; reduced kidney size; haematuria and anaemia.

Conventionally, renal dysfunction is deemed as comprising major classes denoted as acute renal or kidney failure (acute renal or kidney disease or injury, e.g., acute kidney injury or "AKI") or chronic renal or kidney failure (chronic renal or kidney disease). Whereas progression is typically fast (e.g., days to weeks) in acute renal failure, renal failure may be traditionally regarded as chronic if it persists for at least 3 months and its progression may take in the range of years.

Acute renal dysfunction or failure may be staged (classified, graded) into 5 distinct stages using the "RIFLE" (Risk, Injury, Failure, Loss, end-stage renal disease) staging system as set out here below (based on Lameire et al. 2005, Lancet 365: 417-430):

| | | |
|---|---|---|
| Stage | GFR (based on serum creatinine) criteria | Urine output criteria |
| | GFR=glomerular filtration rate | |
| "Risk" | Serum creatinine increased 1.5 times | < 0.5 mL / kg / h for 6 h |
| "Injury" | Serum creatinine increased 2.0 times | < 0.5 mL / kg / h for 12 h |
| "Failure" | Serum creatinine increased 3.0 times, | < 0.3 mL / kg / h for 24 h |
| | or creatinine >355 mM/L when there | or anuria for 12 h |
| | was an acute rise of > 44 mM/L | |
| "Loss" | Persistent acute renal failure > 4 weeks | ---- |
| "End-stage" | End-stage renal disease > 3 months | --- |

Chronic renal dysfunction or failure may be staged (classified, graded) based on GFR as set out here below (based on Levey et al. 2005, Kidney Int 67: 2089-2100):
Stage 1: GFR ≥ 90 mL/min (normal or elevated GFR)
Stage 2: GFR = 60-89 mL/min (mild GFR reduction)
Stage 3: GFR = 30-59 mL/min (moderate GFR reduction)
Stage 4: GFR = 15-29 mL/min (severe GFR reduction)
Stage 5: GFR < 15 mL/min (renal failure)

Other staging methods for renal failure resulting in similar or comparable classifications of different stages of renal failure may be used herein.

The present diagnosis, prediction, prognosis and/or monitoring methods may allow to determine that a subject has or is at risk of having acute or chronic renal failure, such as in particular determine any one of the above-described or comparable stages of acute or chronic renal failure in the subject, and/or may allow to discriminate between said stages in the subject.

The causes of acute renal deterioration may be pre-renal, post-renal and/or intra-renal. Pre-renal causes include lack of sufficient blood supply to the kidneys (i.e., renal hypoperfusion), which in turn may be caused by *inter alia* haemorrhage, massive blood loss, congestive heart failure, decompensated liver cirrhosis (liver cirrhosis with complications such as bleedings, ascites), damaged kidney blood vessels, sepsis or systemic inflammation due to infection. Post-renal causes include obstructions of urine collection systems or extra-renal drainage (i.e., obstructive uropathy), which in turn may be caused by *inter alia* medication interfering with normal bladder emptying, prostate diseases, kidney stones, abdominal malignancy (such as ovarian cancer or colorectal cancer), or obstructed urinary catheter. Intra-renal causes include renal tissue-destroying conditions, such as vasculitis, malignant hypertension, acute glomerulonephritis, acute interstitial nephritis and acute tubular necrosis. They can be caused without limitation by ischemic events (such as, e.g., haemoglobinuria, myoglobinuria and myoloma) or by nephrotoxic substances (such as, e.g., antibiotics, radio contrast agents, uric acid, oxalate and drug induced renal toxicity). Subjects having or being at risk of having the above states, conditions or diseases may have or may be at risk of developing acute renal failure. Hence, the present diagnosis, prediction, prognosis and/or monitoring methods may be preferably employed in such patients.

Causes of chronic renal deterioration may include *inter alia* vascular diseases, such as, e.g., bilateral renal artery stenosis, ischemic nephropathy, haemolytic-uremic syndrome and vasculitis, and further focal segmental nephrosclerosis, glomerulosclerosis, glomerulonephritis, IgA nephritis, diabetic nephropathy, lupus nephritis, polycystic kidney disease, chronic tubulointerstitial nephritis (e.g., drug and/or toxin-induced), renal fibrosis, nephronophthisis, kidney stones, and prostate diseases. Subjects having or being at risk of having the above states, conditions or diseases may have or may be at risk of developing chronic renal failure. Hence, the present diagnosis, prediction, prognosis and/or monitoring methods may be preferably employed in such patients.

Dyspnea (dyspnoea or shortness of breath) is known *per se* and may particularly refer to a common and distressing symptom experienced by subjects as unpleasant or uncomfortable respiratory sensations, that may be more particularly defined as a "subjective experience of breathing discomfort that consists of qualitatively distinct sensations that vary in intensity". Dyspnea may be connected to a range of underlying pathologies.

The terms "heart failure", "acute heart failure" and "chronic heart failure" as used herein carry their respective art-established meanings. By means of further guidance, the term "heart failure" as used herein broadly refers to pathological conditions characterised by an impaired diastolic or systolic blood flow rate and thus insufficient blood flow from the ventricle to peripheral organs.

"Acute heart failure" or also termed "acute decompensated heart failure" may be defined as the rapid onset of symptoms and signs secondary to abnormal cardiac function, resulting in the need for urgent therapy. AHF can present itself acute *de novo* (new onset of acute heart failure in a patient without previously known cardiac dysfunction) or as acute decompensation of CHF.

The cardiac dysfunction may be related to systolic or diastolic dysfunction, to abnormalities in cardiac rhythm, or to preload and afterload mismatch. It is often life threatening and requires urgent treatment. According to established classification, AHF includes several distinct clinical conditions of presenting patients: (I) acute decompensated congestive heart failure, (II) AHF with hypertension/hypertensive crisis, (III) AHF with pulmonary oedema, (IVa) cardiogenic shock / low output syndrome, (IVb) severe cardiogenic shock, (V) high output failure, and (VI) right-sided acute heart failure. For detailed clinical description, classification and diagnosis of AHF, and for summary of further AHF classification systems including the Killip classification, the Forrester classification and the 'clinical severity' classification, refer *inter alia* to Nieminen et al. 2005 ("Executive summary of the guidelines on the diagnosis and treatment of acute heart failure: the Task Force on Acute Heart Failure of the European Society of Cardiology". Eur Heart J 26: 384-416) and references therein.

The term "chronic heart failure" (CHF) generally refers to a case of heart failure that progresses so slowly that various compensatory mechanisms work to bring the disease into equilibrium. Common clinical symptoms of CHF include *inter alia* any one or more of breathlessness, diminishing exercise capacity, fatigue, lethargy and peripheral oedema. Other less common symptoms include any one or more of palpitations, memory or sleep disturbance and confusion, and usually co-occur with one or more of the above recited common symptoms.

Left ventricular hypertrophy (LVH) generally encompasses the thickening of the myocardium of the left ventricle of the heart. LVH may represent a pathological reaction to cardiovascular diseases that increase the afterload (e.g., aortic stenosis or aortic insufficiency) or high blood pressure. LVH may also represent primary hypertrophic cardiomyopathy. LVH diagnosis may be made *inter alia* using echocardiography, using criteria known per se such as the Sokolow-Lyon index, the Cornell voltage criteria, the Romhilt-Estes point score system or other voltage-based criteria.

Cardiac fibrosis generally encompasses abnormal thickening of the heart valves due to inappropriate proliferation of cardiac fibroblasts and the concomitant excessive production of matrix proteins.

By "preeclampsia" (PE or pre-eclampsia) is meant the multi-system disorder that is characterised by hypertension with proteinuria or oedema, or both, glomerular dysfunction, brain oedema, liver oedema, or coagulation abnormalities due to pregnancy or the influence of a recent pregnancy and all complications associated with the disorder. Pre-eclampsia generally occurs after the 20th week of gestation. Pre-eclampsia is generally defined as some combination of the following symptoms:
(1) a systolic blood pressure (BP)>140 mmHg and a diastolic BP>90 mmHg after 20 weeks gestation (generally measured on two occasions, 4-168 hours apart),
(2) new onset proteinuria (1+ by dipstick on urinanalysis, >300 mg of protein in a 24-hour urine collection, or a single random urine sample having a protein/creatinine ratio >0.3), and
(3) resolution of hypertension and proteinuria by 12 weeks postpartum.

Severe pre-eclampsia is generally defined as (1) a diastolic BP>110 mmHg (generally measured on two occasions, 4-168 hours apart) or (2) proteinuria characterised by a measurement of 3.5 g or more protein in a 24-hour urine collection or two random urine specimens with at least 3+ protein by dipstick. In pre-eclampsia, hypertension and proteinuria generally occur within seven days of each other. In severe pre-eclampsia, severe hypertension, severe proteinuria and HELLP syndrome (haemolysis, elevated liver enzymes, low platelets) or eclampsia can occur simultaneously or only one symptom at a time. Occasionally, severe pre-eclampsia can lead to the development of seizures. This severe form of the syndrome is referred to as "eclampsia." Eclampsia can also include dysfunction or damage to several organs or tissues such as the liver (e.g., hepatocellular damage, periportal necrosis) and the central nervous system (e.g., cerebral oedema and cerebral haemorrhage). The aetiology of the seizures is thought to be secondary to the development of cerebral oedema and focal spasm of small blood vessels in the kidney. Preeclampsia is associated with foetal complications such as intrauterine growth retardation (IUGR) and small for gestational age (SGA). By "small for gestational age (SGA)" is meant a foetus whose birth weight is a weight less than 2,500 gm or below the 10th percentile for gestational age according to U.S. tables of birth weight for gestational age by race, parity, and infant sex as defined by World Health Organization (WHO) (Zhang and Bowes 1995, Obstet Gynecol 86: 200-208).

The terms "predicting" or "prediction", "diagnosing" or "diagnosis" and "prognosticating" or "prognosis" are commonplace and well-understood in medical and clinical practice. It shall be understood that the phrase "a method for predicting, diagnosing and/or prognosticating" a given disease or condition may also be interchanged with phrases such as "a method for prediction, diagnosis and/or prognosis" of said disease or condition or "a method for making (or determining or establishing) a prediction, diagnosis and/or prognosis" of said disease or condition, or the like.

By means of further explanation and without limitation, "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, *e.g.,* relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, *i.e.,* that the subject is at risk of having such (*e.g*., the risk is significantly increased vis-à-vis a control subject or subject population). The term "prediction of no" diseases or conditions as taught herein as described herein in a subject may particularly mean that the subject has a 'negative' prediction of such, *i.e.,* that the subject's risk of having such is not significantly increased vis-à-vis a control subject or subject population.

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition and the prospect (*e.g*., the probability, duration, and/or extent) of recovery.

A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period.

A poor prognosis of the diseases or conditions as taught herein may generally encompass anticipation of a substandard recovery and/or unsatisfactorily slow recovery, or to substantially no recovery or even further worsening of such.

The term "subject" or "patient" as used herein typically denotes humans, but may also encompass reference to non-human animals, preferably warm-blooded animals, more preferably mammals, such as, *e.g.,* non-human primates, rodents, canines, felines, equines, ovines, porcines, and the like.

The terms "sample" or "biological sample" as used herein include any biological specimen obtained from a subject. Samples may include, without limitation, whole blood, plasma, serum, red blood cells, white blood cells (*e.g*., peripheral blood mononuclear cells), saliva, urine, stool (*i.e.,* faeces), tears, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, semen and vaginal secretions. Preferred samples may include ones comprising LTBP2 protein in detectable quantities. In preferred embodiments, the sample may be whole blood or a fractional component thereof such as, *e.g*., plasma, serum, or a cell pellet. Preferably the sample is readily obtainable by minimally invasive methods, allowing to remove or isolate said sample from the subject. Samples may also include tissue samples and biopsies, tissue homogenates and the like. Preferably, the sample used to detect LTBP2 levels is blood plasma. Also preferably, the sample used to detect LTBP2 levels is urine. The term "plasma" defines the colorless watery fluid of the blood that contains no cells, but in which the blood cells (erythrocytes, leukocytes, thrombocytes, etc.) are suspended, containing nutrients, sugars, proteins, minerals, enzymes, etc.

A molecule or analyte such as a protein, polypeptide or peptide, or a group of two or more molecules or analytes such as two or more proteins, polypeptides or peptides, is "measured" in a sample when the presence or absence and/or quantity of said molecule or analyte or of said group of molecules or analytes is detected or determined in the sample, preferably substantially to the exclusion of other molecules and analytes.

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, *i.e.,* relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, *e.g*., weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second parameters (*e.g*., first and second quantities) may but need not require to first determine the absolute values of said first and second parameters. For example, a measurement method can produce quantifiable readouts (such as, *e.g*., signal intensities) for said first and second parameters, wherein said readouts are a function of the value of said parameters, and wherein said readouts can be directly compared to produce a relative value for the first parameter vs. the second parameter, without the actual need to first convert the readouts to absolute values of the respective parameters.

As used herein, the term "LTBP2" corresponds to the protein commonly known as latent transforming growth factor beta binding protein 2 (LTBP2), also known as GLC3D, LTBP3, MSTP031, C14orf141, i.e. the proteins and polypeptides commonly known under these designations in the art. The terms encompass such proteins and polypeptides of any organism where found, and particularly of animals, preferably vertebrates, more preferably mammals, including humans and non-human mammals, even more preferably of humans. The terms particularly encompass such proteins and polypeptides with a native sequence, *i.e.,* ones of which the primary sequence is the same as that of LTBP2 found in or derived from nature. A skilled person understands that native sequences of LTBP2 may differ between different species due to genetic divergence between such species. Moreover, the native sequences of LTBP2 may differ between or within different individuals of the same species due to normal genetic diversity (variation) within a given species. Also, the native sequences of LTBP2 may differ between or even within different individuals of the same species due to post-transcriptional or post-translational modifications. Accordingly, all LTBP2 sequences found in or derived from nature are considered "native". The terms encompass LTBP2 proteins and polypeptides when forming a part of a living organism, organ, tissue or cell, when forming a part of a biological sample, as well as when at least partly isolated from such sources. The terms also encompass proteins and polypeptides when produced by recombinant or synthetic means.

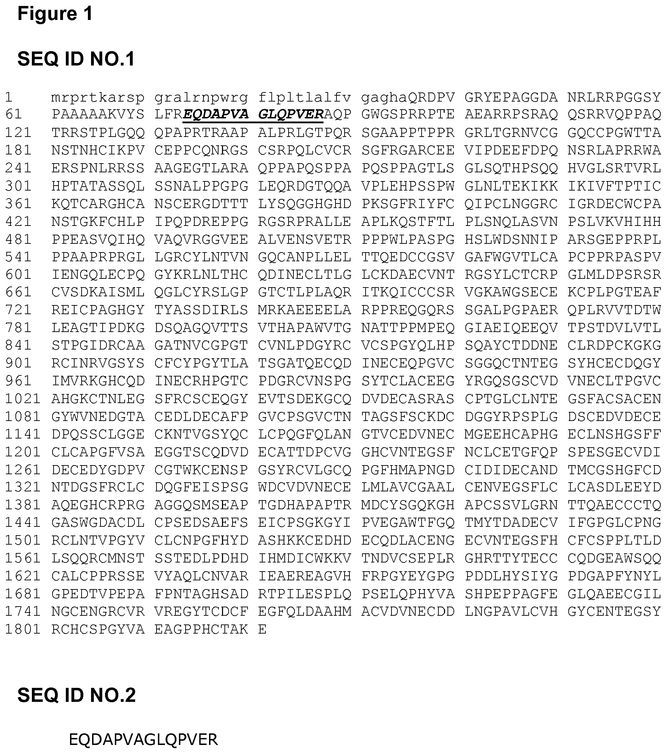

Exemplary LTBP2 includes, without limitation, human LTBP2 having primary amino acid sequence as annotated under NCBI Genbank (http://www.ncbi.nlm.nih.gov/) accession number NP_000419 (sequence version 1) as reproduced in Fig. 1 (SEQ ID NO: 1). A skilled person can also appreciate that said sequences are of precursor of LTBP2 and may include parts which are processed away from mature LTBP2. For example, in Figure 1, an LTBP2 signal peptide is indicated in small caps in the amino acid sequence.

In an embodiment the circulating LTBP2, e.g., secreted form circulating in the blood plasma, may be detected, as opposed to the cell-bound or cell-confined LTBP2 protein. Hence, the reference herein to measuring LTBP2, or to measuring the quantity of LTBP2, may encompass measuring the LTBP2 protein or polypeptide, such as, e.g., measuring the mature and/or the processed soluble/secreted form (e.g. plasma circulating form) of LTBP2 . Preferably, said LTBP2 is a plasma circulating form of LTBP2. The expression "plasma circulating form of LTBP2" or shortly "circulating form" encompasses all LTBP2 proteins that circulate in the plasma, i.e., are not cell- or membrane-bound. Without wanting to be bound by any theory, such circulating forms can be derived from the full-length LTBP2 protein through natural processing, or can be resulting from known degradation processes occurring in said sample. In certain situations, the circulating form can also be the full-length LTBP2 protein, which is found to be circulating in the plasma. Said "circulating form" can thus be any LTBP2 protein or any processed soluble form of LTBP2, that is circulating in the sample, i.e. which is not bound to a cell- or membrane fraction of said sample.

As used herein, the terms "pro-B-type natriuretic peptide" (also abbreviated as "proBNP") and "amino terminal pro-B-type natriuretic peptide" (also abbreviated as "NTproBNP") and "B-type natriuretic peptide" (also abbreviated as "BNP") refer to peptides commonly known under these designations in the art. As further explanation and without limitation, *in vivo* proBNP, NTproBNP and BNP derive from natriuretic peptide precursor B preproprotein (preproBNP). In particular, proBNP peptide corresponds to the portion of preproBNP after removal of the N-terminal secretion signal (leader) sequence from preproBNP. NTproBNP corresponds to the N-terminal portion and BNP corresponds to the C-terminal portion of the proBNP peptide subsequent to cleavage of the latter C-terminally adjacent to amino acid 76 of proBNP.

The term "Cystatin C", also known as ARMD11; MGC117328, Cystatin-3 (CST3), refers to peptides commonly known under these designations in the art, as exemplarily annotated under Genbank accession number NP_000090 (sequence version 1).

As used herein, "neutrophil gelatinase-associated lipocalin" or "NGAL", also known as oncogenic lipocalin 24P3, uterocalin or lipocalin 2 (LCN2), refers to peptides commonly known under these designations in the art, as exemplarily annotated under Genbank accession number NP_005555 (sequence version 2).

The term "C-reactive protein", also known as CRP or PTX1, refers to peptides commonly known under these designations in the art, as exemplarily annotated under Genbank accession number NP_000558 (sequence version 2).

The term "beta-trace protein", also known as *inter alia* prostaglandin-H2 D-isomerase, prostaglandin-D2 synthase, cerebrin-28 and PTGDS, refers to peptides commonly known under these designations in the art, as exemplarily annotated under Genbank accession number NP_000945 (sequence version 3).

The term "kidney injury molecule 1" or KIM-1 refers to peptides commonly known under these designations in the art, as exemplarily disclosed in Ichimura et al. 2004 (Am J Physiol Renal Physiol 286(3): F552-63) and Ichimura et al. 1998 (J Biol Chem 273: 4135-4142).

The term "interleukin-18" refers to peptides commonly known under this designation in the art, as exemplarily annotated under Genbank accession number NP_001553 (sequence version 1).

Unless otherwise apparent from the context, reference herein to any protein, polypeptide or peptide encompasses such from any organism where found, and particularly preferably from animals, preferably vertebrates, more preferably mammals, including humans and non-human mammals, even more preferably from humans.

Further, unless otherwise apparent from the context, reference herein to any protein, polypeptide or peptide and fragments thereof may generally also encompass modified forms of said protein, polypeptide or peptide and fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

In an embodiment, LTBP2 and fragments thereof, or other biomarkers as employed herein and fragments thereof, may be human, *i.e.,* their primary sequence may be the same as a corresponding primary sequence of or present in a naturally occurring human peptides, polypeptides or proteins. Hence, the qualifier "human" in this connection relates to the primary sequence of the respective proteins, polypeptides, peptides or fragments, rather than to their origin or source. For example, such proteins, polypeptides, peptides or fragments may be present in or isolated from samples of human subjects or may be obtained by other means (*e.g*., by recombinant expression, cell-free translation or non-biological peptide synthesis).

The term "fragment" of a protein, polypeptide or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said protein or polypeptide, such as, for example, *in vivo* or *in vitro,* such as, for example, by physical, chemical and/or enzymatic proteolysis. Without limitation, a fragment of a protein, polypeptide or peptide may represent at least about 5%, or at least about 10%, *e.g*., ≥ 20%, ≥ 30% or ≥ 40%, such as ≥ 50%, *e.g., ≥* 60%, ≥ 70% or ≥ 80%, or even ≥ 90% or ≥ 95% of the amino acid sequence of said protein, polypeptide or peptide.

For example, a fragment may include a sequence of ≥ 5 consecutive amino acids, or ≥ 10 consecutive amino acids, or ≥ 20 consecutive amino acids, or ≥ 30 consecutive amino acids, *e.g.,* ≥40 consecutive amino acids, such as for example ≥ 50 consecutive amino acids, *e.g.,* ≥ 60, ≥ 70, ≥ 80, ≥ 90, ≥ 100, ≥ 200, ≥ 300, ≥ 400, ≥ 500 or ≥ 600 consecutive amino acids of the corresponding full length protein.

In an embodiment, a fragment may be N-terminally and/or C-terminally truncated by between 1 and about 20 amino acids, such as, *e.g.,* by between 1 and about 15 amino acids, or by between 1 and about 10 amino acids, or by between 1 and about 5 amino acids, compared to the corresponding mature, full-length protein or its soluble or plasma circulating form. By means of example, proBNP, NTproBNP and BNP fragments useful as biomarkers are disclosed in WO 2004/094460.

In an embodiment, fragments of a given protein, polypeptide or peptide may be achieved by *in vitro* proteolysis of said protein, polypeptide or peptide to obtain advantageously detectable peptide(s) from a sample. For example, such proteolysis may be effected by suitable physical, chemical and/or enzymatic agents, *e.g.,* proteinases, preferably endoproteinases, *i.e.,* protease cleaving internally within a protein, polypeptide or peptide chain. A non-limiting list of suitable endoproteinases includes serine proteinases (EC 3.4.21), threonine proteinases (EC 3.4.25), cysteine proteinases (EC 3.4.22), aspartic acid proteinases (EC 3.4.23), metalloproteinases (EC 3.4.24) and glutamic acid proteinases. Exemplary non-limiting endoproteinases include trypsin, chymotrypsin, elastase, *Lysobacter enzymogenes* endoproteinase Lys-C, *Staphylococcus aureus* endoproteinase Glu-C (endopeptidase V8) or *Clostridium histolyticum* endoproteinase Arg-C (clostripain). Further known or yet to be identified enzymes may be used; a skilled person can choose suitable protease(s) on the basis of their cleavage specificity and frequency to achieve desired peptide forms. Preferably, the proteolysis may be effected by endopeptidases of the trypsin type (EC 3.4.21.4), preferably trypsin, such as, without limitation, preparations of trypsin from bovine pancreas, human pancreas, porcine pancreas, recombinant trypsin, Lys-acetylated trypsin, trypsin in solution, trypsin immobilised to a solid support, *etc.* Trypsin is particularly useful, *inter alia* due to high specificity and efficiency of cleavage. The invention also contemplates the use of any trypsin-like protease, *i.e.,* with a similar specificity to that of trypsin. Otherwise, chemical reagents may be used for proteolysis. For example, CNBr can cleave at Met; BNPS-skatole can cleave at Trp. The conditions for treatment, *e.g.,* protein concentration, enzyme or chemical reagent concentration, pH, buffer, temperature, time, can be determined by the skilled person depending on the enzyme or chemical reagent employed.

Also provided is thus an isolated fragment of LTBP2 as defined here above. Such fragments may give useful information about the presence and quantity of LTBP2 in biological samples, whereby the detection of said fragments is of interest. Hence, the herein disclosed fragments of LTBP2 are useful biomarkers. A preferred LTBP2 fragment may comprise, consist essentially of or consist of the sequence as set forth in SEQ ID NO: 2.

The term "isolated" with reference to a particular component (such as for instance, a protein, polypeptide, peptide or fragment thereof) generally denotes that such component exists in separation from - for example, has been separated from or prepared in separation from - one or more other components of its natural environment. For instance, an isolated human or animal protein, polypeptide, peptide or fragment exists in separation from a human or animal body where it occurs naturally.

The term "isolated" as used herein may preferably also encompass the qualifier "purified". As used herein, the term "purified" with reference to protein(s), polypeptide(s), peptide(s) and/or fragment(s) thereof does not require absolute purity. Instead, it denotes that such protein(s), polypeptide(s), peptide(s) and/or fragment(s) is (are) in a discrete environment in which their abundance (conveniently expressed in terms of mass or weight or concentration) relative to other proteins is greater than in a biological sample. A discrete environment denotes a single medium, such as for example a single solution, gel, precipitate, lyophilisate, *etc.* Purified peptides, polypeptides or fragments may be obtained by known methods including, for example, laboratory or recombinant synthesis, chromatography, preparative electrophoresis, centrifugation, precipitation, affinity purification, *etc.*

Purified protein(s), polypeptide(s), peptide(s) and/or fragment(s) may preferably constitute by weight ≥ 10%, more preferably ≥ 50%, such as ≥ 60%, yet more preferably ≥ 70%, such as ≥ 80%, and still more preferably ≥ 90%, such as ≥ 95%, ≥ 96%, ≥ 97%, ≥ 98%, ≥ 99% or even 100%, of the protein content of the discrete environment. Protein content may be determined, *e.g.,* by the Lowry method (Lowry et al. 1951. J Biol Chem 193: 265), optionally as described by Hartree 1972 (Anal Biochem 48: 422-427). Also, purity of peptides or polypeptides may be determined by SDS-PAGE under reducing or nonreducing conditions using Coomassie blue or, preferably, silver stain.

Further disclosed are isolated LTBP2 or fragments thereof as taught herein comprising a detectable label. This facilitates ready detection of such fragments. The term "label" as used throughout this specification refers to any atom, molecule, moiety or biomolecule that can be used to provide a detectable and preferably quantifiable read-out or property, and that can be attached to or made part of an entity of interest, such as a peptide or polypeptide or a specific-binding agent. Labels may be suitably detectable by mass spectrometric, spectroscopic, optical, colorimetric, magnetic, photochemical, biochemical, immunochemical or chemical means. Labels include without limitation dyes; radiolabels such as ³²P, ³³P ³⁵S ¹²⁵I, ¹³¹I; electron-dense reagents; enzymes (e.g. , horse-radish phosphatise or alkaline phosphatise as commonly used in immunoassays); binding moieties such as biotin-streptavidin; haptens such as digoxigenin; luminogenic, phosphorescent or fluorogenic moieties; mass tags; and fluorescent dyes alone or in combination with moieties that can suppress or shift emission spectra by fluorescence resonance energy transfer (FRET).

For example, the label may be a mass-altering label. Preferably, a mass-altering label may involve the presence of a distinct stable isotope in one or more amino acids of the peptide vis-à-vis its corresponding non-labelled peptide. Mass-labelled peptides are particularly useful as positive controls, standards and calibrators in mass spectrometry applications. In particular, peptides including one or more distinct isotopes are chemically alike, separate chromatographically and electrophoretically in the same manner and also ionise and fragment in the same way. However, in a suitable mass analyser such peptides and optionally select fragmentation ions thereof will display distinguishable m/z ratios and can thus be discriminated. Examples of pairs of distinguishable stable isotopes include H and D, ¹²C and ¹³C, ¹⁴N and ¹⁵N or ¹⁶O and ¹⁸O. Usually, peptides and proteins of biological samples analysed in the present invention may substantially only contain common isotopes having high prevalence in nature, such as for example H, ¹²C, ¹⁴N and ¹⁶O. In such case, the mass-labelled peptide may be labelled with one or more uncommon isotopes having low prevalence in nature, such as for instance D, ¹³C, ¹⁵N and/or ¹⁸O. It is also conceivable that in cases where the peptides or proteins of a biological sample would include one or more uncommon isotopes, the mass-labelled peptide may comprise the respective common isotope(s).

Isotopically-labelled synthetic peptides may be obtained *inter alia* by synthesising or recombinantly producing such peptides using one or more isotopically-labelled amino acid substrates, or by chemically or enzymatically modifying unlabelled peptides to introduce thereto one or more distinct isotopes. By means of example and not limitation, D-labelled peptides may be synthesised or recombinantly produced in the presence of commercially available deuterated L-methionine CH₃-S-CD₂CD₂-CH(NH₂)-COOH or deuterated arginine H₂NC(=NH)-NH-(CD₂)₃-CD(NH₂)-COOH. It shall be appreciated that any amino acid of which deuterated or ¹⁵N- or ¹³C-containing forms exist may be considered for synthesis or recombinant production of labelled peptides. In another non-limiting example, a peptide may be treated with trypsin in H₂ ¹⁶O or H₂ ¹⁸O, leading to incorporation of two oxygens (¹⁶O or ¹⁸O, respectively) at the COOH-termini of said peptide (*e.g.,* US 2006/105415).

Accordingly, also contemplated is the use of LTBP2 and isolated fragments thereof as taught herein, optionally comprising a detectable label, as (positive) controls, standards or calibators in qualitative or quantitative detection assays (measurement methods) of LTBP2, and particularly in such methods for predicting, diagnosing, prognosticating and/or monitoring the diseases or conditions as taught herein in subjects. The proteins, polypeptides or peptides may be supplied in any form, *inter alia* as precipitate, vacuum-dried, lyophilisate, in solution as liquid or frozen, or covalently or non-covalently immobilised on solid phase, such as for example, on solid chromatographic matrix or on glass or plastic or other suitable surfaces (*e.g.,* as a part of peptide arrays and microarrays). The peptides may be readily prepared, for example, isolated from natural sources, or prepared recombinantly or synthetically.

Further disclosed are binding agents capable of specifically binding to any one or more of the isolated fragments of LTBP2 as taught herein. Also disclosed are binding agents capable of specifically binding to only one of isolated fragments of LTBP2 as taught herein. Binding agents as intended throughout this specification may include *inter alia* an antibody, aptamer, photoaptamer, protein, peptide, peptidomimetic or a small molecule.

A binding agent may be capable of binding both the plasma circulating form and the cell-bound or retained from of LTBP2. Preferably, a binding agent may be capable of specifically binding or detecting the plasma circulating form of LTBP2.

The term "specifically bind" as used throughout this specification means that an agent (denoted herein also as "specific-binding agent") binds to one or more desired molecules or analytes, such as to one or more proteins, polypeptides or peptides of interest or fragments thereof substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to protein(s) polypeptide(s), peptide(s) and/or fragment(s) thereof of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

Preferably, the agent may bind to its intended target(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [SBA_T]/[SBA][T], SBA denotes the specific-binding agent, T denotes the intended target. Determination of K_{A} can be carried out by methods known in the art, such as for example, using equilibrium dialysis and Scatchard plot analysis. Specific binding agents as used throughout this specification may include *inter alia* an antibody, aptamer, photoaptamer, protein, peptide, peptidomimetic or a small molecule.

As used herein, the term "antibody" is used in its broadest sense and generally refers to any immunologic binding agent. The term specifically encompasses intact monoclonal antibodies, polyclonal antibodies, multivalent (*e.g.,* 2-, 3- or more-valent) and/or multi-specific antibodies (*e.g.,* bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced in vitro or in vivo.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), *e.g.,* dibodies, tribodies, and multibodies. The above designations Fab, Fab', F(ab')2, Fv, scFv *etc.* are intended to have their art-established meaning.

The term antibody includes antibodies originating from or comprising one or more portions derived from any animal species, preferably vertebrate species, including, *e.g.,* birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius),* Ilama (e.g., *Lama paccos, Lama glama* or *Lama vicugna)* or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA/RNA or any analogue thereof, that can specifically bind to a target molecule such as a peptide. Advantageously, aptamers can display fairly high specificity and affinity *(e.g.,* K_{A} in the order 1×10⁹ M⁻¹) for their targets. Aptamer production is described *inter alia* in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592. The term "photoaptamer" refers to an aptamer that contains one or more photoreactive functional groups that can covalently bind to or crosslink with a target molecule. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134).

The term "small molecule" refers to compounds, preferably organic compounds, with a size comparable to those organic molecules generally used in pharmaceuticals. The term excludes biological macromolecules (*e.g.,* proteins, nucleic acids, etc.). Preferred small organic molecules range in size up to about 5000 Da, *e.g.,* up to about 4000, preferably up to 3000 Da, more preferably up to 2000 Da, even more preferably up to about 1000 Da, *e.g.,* up to about 900, 800, 700, 600 or up to about 500 Da.

Hence, also disclosed are methods for immunising animals, e.g., non-human animals such as laboratory or farm, animals using (i.e., using as the immunising antigen) the herein taught fragments of LTBP2, optionally attached to a presenting carrier. Immunisation and preparation of antibody reagents from immune sera is well-known *per* se and described in documents referred to elsewhere in this specification. The animals to be immunised may include any animal species, preferably warm-blooded species, more preferably vertebrate species, including, *e.g.,* birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel, Ilama or horse. The term "presenting carrier" or "carrier" generally denotes an immunogenic molecule which, when bound to a second molecule, augments immune responses to the latter, usually through the provision of additional T cell epitopes. The presenting carrier may be a (poly)peptidic structure or a non-peptidic structure, such as *inter alia* glycans, polyethylene glycols, peptide mimetics, synthetic polymers, etc. Exemplary non-limiting carriers include human Hepatitis B virus core protein, multiple C3d domains, tetanus toxin fragment C or yeast Ty particles.

Immune sera obtained or obtainable by immunisation as taught herein may be particularly useful for generating antibody reagents that specifically bind to one or more of the herein disclosed fragments of LTBP2.

Further disclosed are methods for selecting specific-binding agents which bind (a) one or more of the LTBP2 fragments taught herein, substantially to the exclusion of (b) LTBP2 and/or other fragments thereof. Conveniently, such methods may be based on subtracting or removing binding agents which cross-react or cross-bind the non-desired LTBP2 molecules under (b). Such subtraction may be readily performed as known in the art by a variety of affinity separation methods, such as affinity chromatography, affinity solid phase extraction, affinity magnetic extraction, etc.

Any existing, available or conventional separation, detection and quantification methods can be used herein to measure the presence or absence (*e.g.,* readout being present vs. absent; or detectable amount vs. undetectable amount) and/or quantity (*e.g.,* readout being an absolute or relative quantity, such as, for example, absolute or relative concentration) of LTBP2 and/or fragments thereof and optionally of the one or more other biomarkers or fragments thereof in samples (any molecules or analytes of interest to be so-measured in samples, including LTBP2 and fragments thereof, may be herein below referred to collectively as biomarkers).

For example, such methods may include immunoassay methods, mass spectrometry analysis methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1 st ed., Humana Press 2000, ISBN 0896037282.

By means of further explanation and not limitation, direct ELISA employs a labelled primary antibody to bind to and thereby quantify target antigen in a sample immobilised on a solid support such as a microwell plate. Indirect ELISA uses a non-labelled primary antibody which binds to the target antigen and a secondary labelled antibody that recognises and allows to quantify the antigen-bound primary antibody. In sandwich ELISA the target antigen is captured from a sample using an immobilised 'capture' antibody which binds to one antigenic site within the antigen, and subsequent to removal of non-bound analytes the so-captured antigen is detected using a 'detection' antibody which binds to another antigenic site within said antigen, where the detection antibody may be directly labelled or indirectly detectable as above. Competitive ELISA uses a labelled 'competitor' that may either be the primary antibody or the target antigen. In an example, non-labelled immobilised primary antibody is incubated with a sample, this reaction is allowed to reach equilibrium, and then labelled target antigen is added. The latter will bind to the primary antibody wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colorimetric, chemiluminescent or fluorescent, magnetic, piezo electric, pyroelectric and other.

Radioimmunoassay (RIA) is a competition-based technique and involves mixing known quantities of radioactively-labelled (e.g., ¹²⁵I- or ¹³¹I-labelled) target antigen with antibody to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821198 for guidance).

Generally, any mass spectrometric (MS) techniques that can obtain precise information on the mass of peptides, and preferably also on fragmentation and/or (partial) amino acid sequence of selected peptides (*e.g.,* in tandem mass spectrometry, MS/MS; or in post source decay, TOF MS), are useful herein. Suitable peptide MS and MS/MS techniques and systems are well-known *per se* (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)ⁿ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)ⁿ; atmospheric pressure photoionization mass spectrometry (APPI-MS); APPI-MS/MS; and APPI- (MS)ⁿ. Peptide ion fragmentation in tandem MS (MS/MS) arrangements may be achieved using manners established in the art, such as, *e.g.,* collision induced dissociation (CID). Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods described herein below.

Chromatography can also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, *etc.*

Chromatography as used herein may be preferably columnar (*i.e.,* wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like.

Chromatography, including single-, two- or more-dimensional chromatography, may be used as a peptide fractionation method in conjunction with a further peptide analysis method, such as for example, with a downstream mass spectrometry analysis as described elsewhere in this specification.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The various aspects and embodiments taught herein may further rely on comparing the quantity of LTBP2 measured in samples with reference values of the quantity of LTBP2, wherein said reference values represent known predictions, diagnoses and/or prognoses of diseases or conditions as taught herein.

For example, distinct reference values may represent the prediction of a risk (e.g., an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values can represent the diagnosis of a given disease or condition as taught herein vs. the diagnosis of no such disease or condition (such as, e.g., the diagnosis of healthy, or recovered from said disease or condition, *etc.).* In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such different between values or profiles being compared. A comparison may include a visual inspection, an arithmetical or statistical comparison of measurements. Such statistical comparisons include, but are not limited to, applying a rule. If the values or biomarker profiles comprise at least one standard, the comparison to determine a difference in said values or biomarker profiles may also include measurements of these standards, such that measurements of the biomarker are correlated to measurements of the internal standards.

Reference values for the quantity of LTBP2 may be established according to known procedures previously employed for other biomarkers.

For example, a reference value of the quantity of LTBP2 for a particular prediction, diagnosis and/or prognosis of given disease or condition as taught herein may be established by determining the quantity of LTBP2 in sample(s) from one individual or from a population of individuals characterised by said particular prediction, diagnosis and/or prognosis of said disease or condition (*i.e.,* for whom said prediction, diagnosis and/or prognosis of renal dysfunction holds true). Such population may comprise without limitation ≥ 2, ≥ 10, ≥ 100, or even several hundreds or more individuals.

Hence, by means of an illustrative example, reference values of the quantity of LTBP2 for the diagnoses of a given disease or condition as taught herein vs. no such disease or condition may be established by determining the quantity of LTBP2 in sample(s) from one individual or from a population of individuals diagnosed (e.g., based on other adequately conclusive means, such as, for example, clinical signs and symptoms, imaging, ECG, *etc.)* as, respectively, having or not having said disease or condition.

In an embodiment, reference value(s) as intended herein may convey absolute quantities of LTBP2. In another embodiment, the quantity of LTBP2 in a sample from a tested subject may be determined directly relative to the reference value (e.g., in terms of increase or decrease, or fold-increase or fold-decrease). Advantageously, this may allow to compare the quantity of LTBP2 in the sample from the subject with the reference value (in other words to measure the relative quantity of LTBP2 in the sample from the subject vis-a-vis the reference value) without the need to first determine the respective absolute quantities of LTBP2.

The expression level or presence of a biomarker in a sample of a patient may sometimes fluctuate, i.e. increase or decrease significantly without change (appearance of, worsening or improving of) symptoms. In such an event, the marker change precedes the change in symptoms and becomes a more sensitive measure than symptom change. Therapeutic intervention can be initiated earlier and be more effective than waiting for deteriorating symptoms. Early intervention at a more benign status may be carried out safely at home, which is a major improvement from treating seriously deteriorated patients in the emergency room.

Measuring the LTBP2 level of the same patient at different time points can in such a case thus enable the continuous monitoring of the status of the patient and can lead to prediction of worsening or improvement of the patient's condition with regard to a given disease or condition as taught herein. A home or clinical test kit or device as indicated herein can be used for this continuous monitoring. One or more reference values or ranges of LTBP2 levels linked to a certain disease state (e.g. renal dysfunction or no renal dysfunction) for such a test can e.g. be determined beforehand or during the monitoring process over a certain period of time in said subject. Alternatively, these reference values or ranges can be established through data sets of several patients with highly similar disease phenotypes, e.g. from healthy subjects or subjects not having teh disease or condition of interest. A sudden deviation of the LTBP2 levels from said reference value or range can predict the worsening of the condition of the patient (e.g. at home or in the clinic) before the (often severe) symptoms actually can be felt or observed.

Also disclosed is thus a method or algorithm for determining a significant change in the level of the LTBP2 marker in a certain patient, which is indicative for change (worsening or improving) in clinical status. In addition, the invention allows establishing the diagnosis that the subject is recovering or has recovered from a given disease or condition as taught herein.

In an embodiment the present methods may include a step of establishing such reference value(s). In an embodiment, the present kits and devices may include means for establishing a reference value of the quantity of LTBP2 for a particular prediction, diagnosis and/or prognosis of a given disease or condition as taught herein. Such means may for example comprise one or more samples (e.g., separate or pooled samples) from one or more individuals characterised by said particular prediction, diagnosis and/or prognosis of said disease or condition.

The various aspects and embodiments taught herein may further entail finding a deviation or no deviation between the quantity of LTBP2 measured in a sample from a subject and a given reference value.

A "deviation" of a first value from a second value may generally encompass any direction (e.g., increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD, or ±1xSE or ±2xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the prediction, diagnosis and/or prognosis methods, e.g., sensitivity and/or specificity of at least 50%, or at least 60%, or at least 70%, or at least 80%, or at least 85%, or at least 90%, or at least 95%.

For example, in an embodiment, an elevated quantity of LTBP2 in the sample from the subject - preferably at least about 1.1-fold elevated, or at least about 1.2-fold elevated, more preferably at least about 1.3-fold elevated, even more preferably at least about 1.4-fold elevated, yet more preferably at least about 1.5-fold elevated, such as between about 1.1-fold and 3-fold elevated or between about 1.5-fold and 2-fold elevated - compared to a reference value representing the prediction or diagnosis of no given disease or condition as taught herein or representing a good prognosis for said disease or condition indicates that the subject has or is at risk of having said disease or condition or indicates a poor prognosis for the disease or condition in the subject.

When a deviation is found between the quantity of LTBP2 in a sample from a subject and a reference value representing a certain prediction, diagnosis and/or prognosis of a given disease or condition as taught herein, said deviation is indicative of or may be attributed to the conclusion that the prediction, diagnosis and/or prognosis of said disease or condition in said subject is different from that represented by the reference value.

When no deviation is found between the quantity of LTBP2 in a sample from a subject and a reference value representing a certain prediction, diagnosis and/or prognosis of a given disease or condition as taught herein, the absence of such deviation is indicative of or may be attributed to the conclusion that the prediction, diagnosis and/or prognosis of said disease or condition in said subject is substantially the same as that represented by the reference value.

The above considerations apply analogously to biomarker profiles.

When two or more different biomarkers are determined in a subject, their respective presence, absence and/or quantity may be together represented as a biomarker profile, the values for each measured biomarker making a part of said profile. As used herein, the term "profile" includes any set of data that represents the distinctive features or characteristics associated with a condition of interest, such as with a particular prediction, diagnosis and/or prognosis of a given disease or condition as taught herein. The term generally encompasses *inter alia* nucleic acid profiles, such as for example genotypic profiles (sets of genotypic data that represents the genotype of one or more genes associated with a condition of interest), gene copy number profiles (sets of gene copy number data that represents the amplification or deletion of one or more genes associated with a condition of interest), gene expression profiles (sets of gene expression data that represents the mRNA levels of one or more genes associated with a condition of interest), DNA methylation profiles (sets of methylation data that represents the DNA methylation levels of one or more genes associated with a condition of interest), as well as protein, polypeptide or peptide profiles, such as for example protein expression profiles (sets of protein expression data that represents the levels of one or more proteins associated with a condition of interest), protein activation profiles (sets of data that represents the activation or inactivation of one or more proteins associated with a condition of interest), protein modification profiles (sets of data that represents the modification of one or more proteins associated with a condition of interest), protein cleavage profiles (sets of data that represent the proteolytic cleavage of one or more proteins associated with a condition of interest), as well as any combinations thereof.

Biomarker profiles may be created in a number of ways and may be the combination of measurable biomarkers or aspects of biomarkers using methods such as ratios, or other more complex association methods or algorithms (e.g., rule-based methods). A biomarker profile comprises at least two measurements, where the measurements can correspond to the same or different biomarkers. A biomarker profile may also comprise at least three, four, five, 10, 20, 30 or more measurements. In one embodiment, a biomarker profile comprises hundreds, or even thousands, of measurements.

Hence, for example, distinct reference profiles may represent the prediction of a risk (e.g., an abnormally elevated risk) of having a given disease or condition vs. the prediction of no or normal risk of having said disease or condition. In another example, distinct reference profiles may represent predictions of differing degrees of risk of having said disease or condition.

In a further example, distinct reference profiles can represent the diagnosis of a given disease or condition as taught herein vs. the diagnosis no such disease or condition (such as, e.g., the diagnosis of healthy, recovered from said disease or condition, *etc.).* In another example, distinct reference profiles may represent the diagnosis of said disease or condition of varying severity.

In a yet another example, distinct reference profiles may represent a good prognosis for a disease or condition as taught herein vs. a poor prognosis for said disease or condition. In a further example, distinct reference profiles may represent varyingly favourable or unfavourable prognoses for such disease or condition.

Reference profiles used herein may be established according to known procedures previously employed for other biomarkers.

For example, a reference profile of the quantity of LTBP2 and the presence or absence and/or quantity of one or more other biomarkers for a particular prediction, diagnosis and/or prognosis of a given disease or condition as taught herein may be established by determining the profile in sample(s) from one individual or from a population of individuals characterised by said particular prediction, diagnosis and/or prognosis of said disease or condition (*i.e.,* for whom said prediction, diagnosis and/or prognosis of said disease or condition holds true). Such population may comprise without limitation ≥ 2, ≥ 10, ≥ 100, or even several hundreds or more individuals.

Hence, by means of an illustrative example, reference profiles for the diagnoses of a given disease or condition as taught herein vs. no such disease or condition may be established by determining the biomarker profiles in sample(s) from one individual or from a population of individuals diagnosed as, respectively, having or not having said disease or condition.

In an embodiment the present methods may include a step of establishing such reference profile(s). In an embodiment, the present kits and devices may include means for establishing a reference profile for a particular prediction, diagnosis and/or prognosis of a given disease or condition as taught herein. Such means may for example comprise one or more samples (e.g., separate or pooled samples) from one or more individuals characterised by said particular prediction, diagnosis and/or prognosis of said disease or condition.

Further, art-known multi-parameter analyses may be employed *mutatis mutandis* to determine deviations between groups of values and profiles generated there from (e.g., between sample and reference biomarker profiles).

When a deviation is found between the sample profile and a reference profile representing a certain prediction, diagnosis and/or prognosis of a given disease or condition as taught herein, said deviation is indicative of or may be attributed to the conclusion that the prediction, diagnosis and/or prognosis of said disease or condition in said subject is different from that represented by the reference profile.

When no deviation is found between the sample profile and a reference profile representing a certain prediction, diagnosis and/or prognosis of a given disease or condition as taught herein, the absence of such deviation is indicative of or may be attributed to the conclusion that the prediction, diagnosis and/or prognosis of said disease or condition in said subject is substantially the same as that represented by the reference profile.

The present invention further provides kits or devices for diagnosing, predicting, prognosticating and/or monitoring of any one disease or condition as taught herein comprising means for detecting the level of the LTBP2 marker in a sample of the patient. In a more preferred embodiment, such a kit or kits of the invention can be used in clinical settings or at home. The kit according to the invention can be used for diagnosing said disease or condition, for monitoring the effectiveness of treatment of a subject suffering from said disease or condition with an agent, or for preventive screening of subjects for the occurrence of said disease or condition in said subject.

In a clinical setting, the kit or device can be in the form of a bed-side device or in an emergency team setting, e.g. as part of the equipment of an ambulance or other moving emergency vehicle or team equipment or as part of a first-aid kit. The diagnostic kit or device can assist a medical practitioner, a first aid helper, or nurse to decide whether the patient under observation is developing an acute heart failure, after which appropriate action or treatment can be performed.

A home-test kit gives the patient a readout which he can communicate to a medicinal practitioner, a first aid helper or to the emergency department of a hospital, after which appropriate action can be taken. Such a home-test device is of particular interest for people having either a history of, or are at risk of suffering from any one disease or condition as taught herein or have a history or are at risk of suffering from dyspnea. Such subjects with a high risk for a disease or condition as taught herein or having a history of dyspnea could certainly benefit from having a home test device or kit according to the invention at home, *inter alia* because they can then easily distinguish between a renal dysfunction event and another event causing the dyspnea, resulting in an easier way of determining the actions to be taken to resolve the problem.

Typical kits or devices according to the invention comprise the following elements:
a) a means for obtaining a sample from the subject
b) a means or device for measuring the amount of the LTBP2 marker in said sample and visualizing whether the amount of the LTBP2 marker in said sample is below or above a certain threshold level or value, indicating whether the subject is suffering from a given disease or condition as taught herein or not.

In any of the embodiments of the invention, the kits or devices can additionally comprise c) means for communicating directly with a medical practitioner, an emergency department of the hospital or a first aid post, indicating that a person is suffering from said disease or condition or not.

The term "threshold level or value" or "reference value" is used interchangeably as a synonym and is as defined herein. It can also be a range of base-line (e.g. "dry weight") values determined in an individual patient or in a group of patients with highly similar disease conditions.

In any of the embodiments of the invention, the device or kit or kits of the invention can additionally comprise means for detecting the level of an additional marker in the sample of said patient. Additional markers could for example be creatinine, Cystatin C, NGAL, beta-trace protein, kidney injury molecule 1 (KIM-1), interleukin-18 (IL-18), BNP, proBNP, NTproBNP and CRP, and fragments or precursors of any one thereof.

Any of kits as defined herein can be used as a bed-side device for use by the subject himself or by a clinical practitioner.

In said kit of the invention, the means for obtaining a sample from the subject (a) can be any means for obtaining a sample from the subject known in the art. Examples for obtaining e.g. a blood sample are known in the art and could be any kind of finger or skin prick or lancet based device, which basically pierces the skin and results in a drop of blood being released from the skin. When a urine sample is used, the means for obtaining a sample from the subject can be in the form of an absorbent strip such as the ones used in home pregnancy tests known in the art. In analogy, a saliva sample could be obtained using a mount swab known in the art. Example of blood sampling devices or other sampling devices are for example given in U.S. Pat. No. 4,802,493, 4,966,159, 5,099,857, 6,095,988, 5,944,671, 4,553,541, 3,760,809, 5,395,388, 5,212,879, 5,630,828, 5,133,730, 4,653,513, 5,368,047, 5,569,287, 4,360,016, 5,413,006 and U.S. Pat. Applic. 2002/111565, 2004/0096959, 2005/143713, 2005/137525, 2003/0153900, 2003/0088191, WO9955232, WO2005/049107, WO2004/060163, WO02/056751, WO02/100254, WO2003/022330, WO2004/066822, WO97/46157, WO2004/039429, or EP0364621, EP0078724, EP1212138, EP0081975, or EP0292928. The way of providing or obtaining the sample is by no means limiting.

In said kit of the invention, the means or device for measuring the amount of the LTBP2 marker in said sample (b) can be any means or device that can specifically detect the amount of the LTBP2 protein in the sample. Examples are systems comprising LTBP2 specific binding molecules attached to a solid phase, e.g. lateral flow strips or dipstick devices and the like well known in the art. One non-limiting example to perform a biochemical assay is to use a test-strip and labelled antibodies which combination does not require any washing of the membrane. The test strip is well known, for example, in the field of pregnancy testing kits where an anti-hCG antibody is present on the support, and is carried complexed with hCG by the flow of urine onto an immobilised second antibody that permits visualisation. Other non-limiting examples of such home test devices, systems or kits can be found for example in the following U.S. patents: 6,107,045, US6,974,706, 5,108,889, 6,027,944, 6,482,156, 6,511,814, 5,824,268, 5,726,010, 6,001,658 or U.S. patent applications: 2008/0090305 or 2003/0109067.

In a preferred embodiment, the invention provides a lateral flow device or dipstick. Such dipstick comprises a test strip allowing migration of a sample by capillary flow from one end of the strip where the sample is applied to the other end of such strip where presence of an analyte in said sample is measured.

In another embodiment, the invention provides a device comprising a reagent strip. Such reagent strip comprises one or more test pads which when wetted with the sample, provide a color change in the presence of an analyte and/or indicate the concentration of the protein in said sample.

In one preferred embodiment of the kit of the invention, the means or device (1) for measuring the amount of protein in a sample (b) is a solid support (7) having a proximal (2) and distal (3) end, comprising:
- a sample application zone (4) in the vicinity of the proximal end,
- a reaction zone (5) distal to the sample application zone (4), and
- a detection zone (6) distal to the reaction zone (5),
   whereby said support has a capillary property that directs a flow of fluid sample applied in the application zone in a direction from the proximal end to the distal end,
- optionally, the means or device also comprises a source of fluid, e.g. in a container, dropper pipette or vial, enabling viscous samples to flow easier through the strip.

The reaction zone (5) comprises one or more bands (10) of LTBP2 binding molecule conjugated to a detection agent (e.g. colloidal gold) which LTBP2 binding molecule conjugate is disposed on the solid support such that it can migrate with the capillary flow of fluid *i.e.* it is not immobilised. The detection zone (6) comprises one or more capture bands (11) comprising a population of LTBP2 binding molecules immobilised on the solid support.

When a sample is applied to the sample application zone (4), it migrates towards the reaction zone (5) by capillary flow. Any LTBP2 present in the sample reacts with the LTBP2 labelled binding molecule conjugate, and the complex so formed is carried by capillary flow to the detection zone (6). The detection zone (6), having LTBP2 binding molecules permanently immobilised thereon, captures and immobilises any complex, resulting in a localised concentration of conjugate that can be visualised.

The two zones (5 and 6) as described herein (one zone with the non-fixed conjugates and one zone with the fixed capture antibodies) generally do not overlap. They may be adjacently arranged with an absence or presence of an intervening gap of solid support devoid of band. A band may be disposed on a solid support by any means, for example, absorbed, adsorbed, coated, covalently attached or dried, depending on whether the reagent is required to be mobilised or not.

In order to obtain a semi-quantitative test strip in which only a signal is formed once the LTBP2 protein level in the sample is higher than a certain predetermined threshold level or value, the reaction zone (5) comprising the non-fixed conjugated LTBP2 binding molecules, could also comprise a predetermined amount of fixed LTBP2 capture antibodies. This enables to capture away a certain amount of LTBP2 protein present in the sample, corresponding to the threshold level or value as predetermined. The remaining amount of LTBP2 protein (if any) bound by the conjugated or labelled binding molecules can then be allowed to migrate to the detection zone (6). In this case, the reaction zone (6) will only receive labelled binding molecule- LTBP2 complexes and subsequently only produce a signal if the level of the LTBP2 protein in the sample is higher than the predetermined threshold level or value.

Another possibility to determine whether the amount of the LTBP2 protein in the sample is below or above a certain threshold level or value, is to use a primary capturing antibody capturing all LTBP2 protein present in the sample, in combination with a labeled secondary antibody, developing a certain signal or color when bound to the solid phase. The intensity of the color or signal can then either be compared to a reference color or signal chart indicating that when the intensity of the signal is above a certain threshold signal, the test is positive (i.e. renal dysfunction or kidney failure is imminent). Alternatively, the amount or intensity of the color or signal can be measured with an electronic device comprising e.g. a light absorbance sensor or light emission meter, resulting in a numerical value of signal intensity or color absorbance formed, which can then be displayed to the subject in the form of a negative result if said numerical value is below the threshold value or a positive result if said numerical value is above the threshold value. This embodiment is of particular relevance in monitoring the LTBP2 level in a patient over a period of time.

The reference value or range can e.g. be determined using the home device in a period wherein the subject is free of a given disease or condition, giving the patient an indication of his base-line LTBP2 level. Regularly using the home test device will thus enable the subject to notice a sudden change in LTBP2 levels as compared to the base-line level, which can enable him to contact a medical practitioner.

Alternatively, the reference value can be determined in the subject suffering from a given disease or condition as taught herein, which then indicates his personal LTBP2 "risk level", i.e. the level of LTBP2 which indicates he is or will soon be exposed to said disease or condition. This risk level is interesting for monitoring the disease progression or for evaluating the effect of the treatment. Reduction of the LTBP2 level as compared to the risk level indicates that the condition of the patient is improving.

Furthermore, the reference value or level can be established through combined measurement results in subjects with highly similar disease states or phenotypes (e.g. all having no disease or condition as taught herein or having said disease or condition).

Non-limiting examples of such semi-quantitative tests known in the art, the principle of which could be used for the home test device according to the present invention are the HIV/AIDS test or Prostate Cancer tests sold by Sanitoets. The home prostate test is a rapid test intended as an initial semi-quantitative test to detect PSA blood levels higher than 4 ng/ml in whole blood. The typical home self-test kit comprises the following components: a test device to which the blood sample is to be administered and which results in a signal when the protein level is above a certain threshold level, an amount of diluent e.g. in dropper pipette to help the transfer of the analytes (i.e. the protein of interest) from the sample application zone to the signal detection zone, optionally an empty pipette for blood specimen collection, a finger pricking device, optionally a sterile swab to clean the area of pricking and instructions of use of the kit.

Similar tests are also known for e.g. breast cancer detection and CRP-protein level detection in view of cardiac risk home tests. The latter test encompasses the sending of the test result to a laboratory, where the result is interpreted by a technical or medical expert. Such telephone or internet based diagnosis of the patient's condition is of course possible and advisable with most of the kits, since interpretation of the test result is often more important than conducting the test. When using an electronic device as mentioned above which gives a numerical value of the level of protein present in the sample, this value can of course easily be communicated through telephone, mobile telephone, satellite phone, E-mail, internet or other communication means, warning a hospital, a medicinal practitioner or a first aid team that a person is, or may be at risk of, suffering from kidney failure. A non-limiting example of such a system is disclosed in U.S. patent 6,482,156.

Reference is made in the description below to the drawings which exemplify particular embodiments of the invention; they are not at all intended to be limiting. The skilled person may adapt the device and substituent components and features according to the common practices of the person skilled in the art.

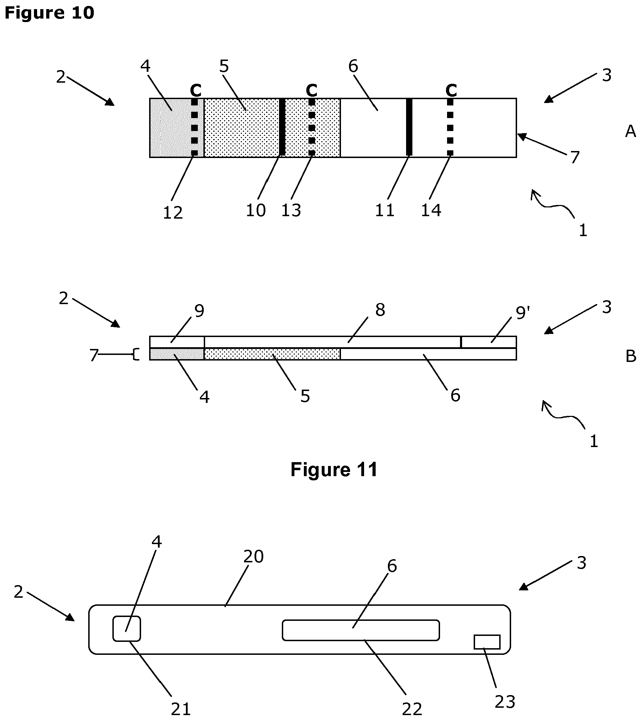

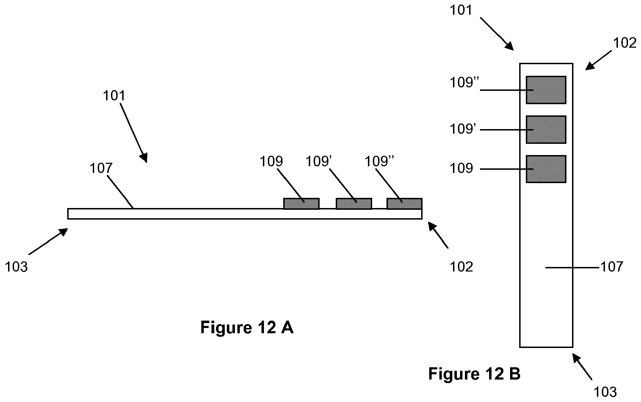

Figure 10A and B shows a preferred embodiment of a test strip of the invention. The strip **(1)** includes a proximal end **(2)** and a distal end **(3).** A sample application zone **(4)** is provided in the proximal end **(2),** a reaction zone **(5)** is adjacent thereto and a detection zone **(6)** is in the vicinity of the distal end **(3).** A sample may be deposited onto the solid support **(7)** at the application zone **(4)** to transfer by capillary action to the detection zone **(6).** A protective layer **(8)** that covers either or both the surfaces of the solid support **(7),** except for a region of the sample application zone **(4)** may be provided. Such protective layer protects the sample and chemical constituency of the strip from contamination and evaporation. One or more absorbent pads **(9)** in capillary contact with the sample application zone **(4)** of the solid support **(7)** may absorb and release sample as necessary; such pad **(9)** is typically placed on the surface of the solid support **(7)** that is the same or opposing the sample application zone **(4).** In Figure 12B, the absorbent pad **(9)** is part of the sample application zone **(4).** One or more other absorbent pads **(9')** in capillary may be placed in contact with the detection zone **(6)** of the solid support **(7),** distal to any capture bands **(11), (14).** These pads **(9')** may absorb fluid that has passed through the solid support; such pad **(9')** is typically placed on the surface of the solid support (7) that is the same or opposing the sample application zone **(4).** The solid support (7) may made from any suitable material that has a capillary action property, and may have the same properties as described above. It should also be capable of supporting a substance (e.g. non-immobilised LTBP2 binding molecule), which, when hydrated, can migrate across the solid support by a capillary action fluid flow.

The solid support **(7)** may also comprise a band of LTBP2 binding molecule conjugate **(10),** located in the reaction zone **(5),** at a position distal to the sample application zone **(4).** Any LTBP2 in the sample is carried by capillary action towards this band **(10),** where it reacts with the permanently immobilised LTBP2 binding molecule conjugate.

The LTBP2 binding molecule conjugate may be associated with or attached to a detection agent to facilitate detection. Examples of lab detection agents include, but are not limited to, luminescent labels; colorimetric labels, such as dyes; fluorescent labels; or chemical labels, such as electroactive agents (*e.g.*, ferrocyanide); enzymes; radioactive labels; or radiofrequency labels. More commonly, the detection agent is a particle. Examples of particles useful in the practice of the invention include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles are colloidal gold particles. Colloidal gold may be made by any conventional means, such as the methods outlined in G. Frens, 1973 Nature Physical Science, 241:20 (1973). Alternative methods may be described in U.S. Pat. Nos. 5,578,577, 5,141,850; 4,775,636; 4,853,335; 4,859,612; 5,079,172; 5,202,267; 5,514,602; 5,616,467; 5,681,775.

The solid support **(7)** further comprises one or more capture bands **(11)** in the detection zone **(6).** A capture band comprises a population of LTBP2 binding molecule permanently immobilised thereon. The LTBP2: LTBP2-binding molecule conjugate complex formed in the reaction zone **(5)** migrates towards the detection zone **(6)** where said band **(11)** captures migrating complex, and concentrates it, allowing it to be visualised either by eye, or using a machine reader. The LTBP2 binding molecule present in the reaction zone **(5)** and in the detection zone **(6)** may reaction to the same part of LTBP2 or may react to different parts of LTBP2.

One or more controls bands **(12)** may be present on the solid support **(7).** For example, a non-immobilised peptide **(12)** might be present in the sample application zone **(4),** which peptide does not cross-react with any of bands of LTBP2 binding molecule **(13) or (14).** As the sample is applied, it migrates towards the reaction zone **(5),** where an anti-peptide antibody conjugate is disposed **(13),** and where a complex peptide-antibody complex is formed. Said complex migrates towards the detection zone **(6),** where a capture band **(14)** of anti-peptide antibody is immobilised on the solid support, and which concentrates said complex enabling visualisation. The control capture band **(14)** is located separately from the LTBP2 capture band **(11),** therefore, a positive reaction can be seen distinct from the detection reaction if the assay is working correctly.

A particular advantage of a control according to the invention is that they are internal controls - that is, the control against which the LTBP2 measurement results may be compared is present on the individual solid support. Therefore, the controls according to the invention may be used to correct for variability in the solid support, for example. Such correction would be impractical with external controls that are based, for example, on a statistical sampling of supports. Additionally, lot-to-lot, and run-to-run, variations between different supports may be minimized by use of control binding agents and control agents according to the invention. Furthermore, the effects of non-specific binding may be reduced. All of these corrections would be difficult to accomplish using external, off-support, controls.

During the assay, LTBP2 from the sample and the LTBP2 binding molecule conjugate combine and concentrate on the solid support **(7).** This combination results in a concentration of compounds that may can be visualised above the background colour of the solid support **(7).** The compounds may be formed from a combination of above-mentioned compounds, including antibodies, detection agents, and other particles associated with the reaction and detection zones. Based on the particular assay being performed, the reaction and detection zones may be selectively implemented to achieve an appropriate dynamic range which may be linear or non-linear.

A solid support **(7)** for performing the assay may be housed within the cartridge **(20)** as shown, for example, in Figure 11. The cartridge is preferably watertight, except for one or more openings. The solid support **(7)** may be exposed through an opening **(21)** in the cartridge to provide an application zone **(4)** in proximal end **(2),** and another opening **(22)** to enable reading of detection zone **(6)** close to the distal end **(3).** Cartridge **(20)** may include a sensor code **(23)** for communicating with a reading device.

The presence and/or concentration of LTBP2 in a sample can be measured by surface plasmon resonance (SPR) using a chip having LTBP2 binding molecule immobilized thereon, fluorescence resonance energy transfer (FRET), bioluminescence resonance energy transfer (BRET), fluorescence quenching, fluorescence polarization measurement or other means known in the art. Any of the binding assays described can be used to determine the presence and/or concentration of LTBP2 in a sample. To do so, LTBP2 binding molecule is reacted with a sample, and the concentration of LTBP2 is measured as appropriate for the binding assay being used. To validate and calibrate an assay, control reactions using different concentrations of standard LTBP2 and/or LTBP2 binding molecule can be performed. Where solid phase assays are employed, after incubation, a washing step is performed to remove unbound LTBP2. Bound, LTBP2 is measured as appropriate for the given label (e.g., scintillation counting, fluorescence, antibody-dye etc.). If a qualitative result is desired, controls and different concentrations may not be necessary. Of course, the roles of LTBP2 and LTBP2 binding molecule may be switched; the skilled person may adapt the method so LTBP2 binding molecule is applied to sample, at various concentrations of sample.

A LTBP2 binding molecule according to the invention is any substance that binds specifically to LTBP2. Examples of a LTBP2 binding molecule useful according to the present invention, includes, but is not limited to an antibody, a polypeptide, a peptide, a lipid, a carbohydrate, a nucleic acid, peptide-nucleic acid, small molecule, small organic molecule, or other drug candidate. A LTBP2 binding molecule can be natural or synthetic compound, including, for example, synthetic small molecule, compound contained in extracts of animal, plant, bacterial or fungal cells, as well as conditioned medium from such cells. Alternatively, LTBP2 binding molecule can be an engineered protein having binding sites for LTBP2. According to an aspect of the invention, a LTBP2 binding molecule binds specifically to LTBP2 with an affinity better than 10⁻⁶ M. A suitable LTBP2 binding molecule e can be determined from its binding with a standard sample of LTBP2. Methods for determining the binding between LTBP2 binding molecule and LTBP2 are known in the art. As used herein, the term antibody includes, but is not limited to, polyclonal antibodies, monoclonal antibodies, humanised or chimeric antibodies, engineered antibodies, and biologically functional antibody fragments (e.g. scFv, nanobodies, Fv, etc) sufficient for binding of the antibody fragment to the protein. Such antibody may be commercially available antibody against LTBP2, such as, for example, a mouse, rat, human or humanised monoclonal antibody.

In a preferred embodiment, the binding molecule or agent is capable of binding both the mature membrane- or cell-bound LTBP2 protein or fragment. In a more preferred embodiment, the binding agent or molecule is specifically binding or detecting the soluble form, preferably the plasma circulating form of LTBP2, as defined herein.

According to one aspect of the invention, the LTBP2 binding molecule is labelled with a tag that permits detection with another agent (e.g. with a probe binding partner). Such tags can be, for example, biotin, streptavidin, his-tag, myc tag, maltose, maltose binding protein or any other kind of tag known in the art that has a binding partner. Example of associations which can be utilised in the probe:binding partner arrangement may be any, and includes, for example biotin:streptavidin, his-tag:metal ion (e.g. Ni²⁺), maltose:maltose binding protein.

In another embodiment, the invention provides a simple and accurate colorimetric reagent strip and method for measuring presence of LTBP2 in a sample. More in particular, the present invention also relates to a device comprising a reagent strip. The present reagent strip comprises a solid support which is provided with at least one test pad for measuring the presence of LTBP2 in a sample. Said test pad preferably comprises a carrier matrix incorporating a reagent composition capable of interacting with LTBP2 to produce a measurable response, preferably a visually or instrumentally measurable response. The reagent strip may be manufactured in any size and shape, but in general the reagent strip is longer than wide. The solid support may be composed of any suitable material and is preferably made of firm or stiff material such as cellulose acetate, polyethylene terephthalate, polypropylene, polycarbonate or polystyrene. In general, the carrier matrix is an absorbent material that allows the urine sample to move, in response to capillary forces, through the carrier matrix to contact the reagent composition and produce a detectable or measurable color transition. The carrier matrix can be any substance capable of incorporating the chemical reagents required to perform the assay of interest, as long as the carrier matrix is substantially inert with respect to the chemical reagents, and is porous or absorbent relative to the soluble components of the liquid test sample. The expression "carrier matrix" refers to either bibulous or nonbibulous matrices that are insoluble in water and other physiological fluids and maintain their structural integrity when exposed to water and other physiological fluids. Suitable bibulous matrices include filter paper, sponge materials, cellulose, wood, woven and nonwoven fabrics and the like. Nonbibulous matrices include glass fiber, polymeric films, and preformed or microporous membranes. Other suitable carrier matrices include hydrophilic inorganic powders, such as silica gel, alumina, diatomaceous earth and the like; argillaceous substances; cloth; hydrophilic natural polymeric materials, particularly cellulose material, like cellulosic beads, and especially fibercontaining papers such as filter paper or chromatographic paper; synthetic or modified naturally-occuring polymers, such as crosslinked gelatin, cellulose acetate, polyvinyl chloride, polyacrylamide, cellulose, polyvinyl alcohol, polysulfones, polyesters, polyacrylates, polyurethanes, crosslinked dextran, agarose, and other such crosslinked and noncrosslinked water-insoluble hydrophilic polymers. Hydrophobic and nonabsorptive substances are not suitable for use as the carrier matrix of the present invention. The carrier matrix can be of different chemical compositions or a mixture of chemical compositions. The matrix also can vary in regards to smoothness and roughness combined with hardness and softness. However, in every instance, the carrier matrix comprises a hydrophilic or absorptive material. The carrier matrix is most advantageously constructed from bibulous filter paper or nonbibulous polymeric films. A preferred carrier matrix is a hydrophilic, bibulous matrix, including cellulosic materials, such as paper, and preferably filter paper or a nonbibulous matrix, including polymeric films, such as a polyurethane or a crosslinked gelatin. A reagent composition which produces a colorimetric change when reacted with LTBP2 in a sample can be homogeneously incorporated into the carrier matrix, and the carrier matrix then holds the reagent composition homogeneously throughout the carrier matrix while maintaining carrier matrix penetrability by the predetermined component of the test sample. Examples of suitable reagent compositions may include for instance a LTBP2 binding molecule in case of an antibody-based technique, or pH buffer in case of enzymatic detection. The reagent composition is preferably dried and stabilized onto a test pad adhered to at least one end of a solid support. The test pad onto which the reagent composition is absorbed and dried, is preferably made of a membrane material that shows minimal background color. Preferably, the test pad may be constructed of acid or base washed materials in order to minimize background color. In another embodiment the reagent composition which is dried onto the reagent strip further comprises wetting agents to reduce brittleness of the test pad. Non-limiting examples of preferred wetting agents include TritonX-100, Bioterg, glycerol, 0 Tween, and the like. The reagent composition can be applied to the reagent strip by any method known in the art. For example, the carrier matrix from which the test pads are made may be dipped into a solution of the reagent composition and dried according to techniques known in the art. A reagent strip according to the invention may be provided with multiple test pads to assay for more than one analyte in a urine sample. A reagent strip may be provided comprising a solid support provided with one or more test pads including test pads for measuring the presence of one or more analytes selected from the group comprising proteins such as renal dysfunction markers cystatin C, NGAL, beta-trace protein, kidney injury molecule 1 (KIM-1), interleukin-18 (IL-18), BNP, NT-pro-BNP or fragments thereof, blood, leukocytes, nitrite, glucose, ketones, creatinine, albumin, bilirubin, urobilinogen and/or a pH test pad, and/or a test pad for measuring specific gravity.

A possible embodiment of a reagent strip **101** according to the invention is depicted diagrammatically in **Figure 12 A-B.** The strip **101** includes a proximal end **102** and a distal end **103.** Various test pads **109, 109', 109"** on which the reagent compositions are provided at the proximal end **102** on a solid support **107** of the reagent strip. The strip must be designed in such a way that it can be wetted with a sufficiently large amount of sample, optionally diluted by a physiological fluid improving the capillary flow of a viscous sample such as blood or saliva and the like.

A reagent strip as defined herein is used as follows. Briefly, one or more test pad areas of the reagent strip of the invention is dipped into a sample or a small amount of sample is applied to the reagent strip onto the test pad area(s). A color development which can be analyzed visually or by reflectometry occurs on the reagent strip within a short time, usually within 0.5 to 10 minutes. The change in color of the reagent area on the test pad upon reacting with LTBP2 is preferably directly proportional to the concentration of LTBP2 in the patient sample. The color intensity that develops on the test pad may be determined visually or by a reflectance-based reader, for example. Color development at the test pad area(s) is compared to a reference color or colors to determine an estimate of the amount of LTBP2 present in the sample The color intensity that develops on the test pad is compared to at least one, and preferably at least two standard color shades that correspond to a range of LTBP2 concentration determined by application of a correction factor.

The reagent strip may further comprises a fluorescent or infrared dye, applied either to the support strip or incorporated into a test pad, which ensures proper alignment of the reagent strip in an apparatus having a detection system for the detectable or measurable response.

In another embodiment, the invention also relates to a test pad for measuring the presence of LTBP2 in a sample. Preferably said test pad comprises a carrier matrix incorporating a reagent composition capable of interacting with LTBP2 to produce a measurable response, preferably a visually or instrumentally measurable response. In another preferred embodiment the invention provides a test pad according as define herein for use in on a reagent strip, preferably on a reagent strip as defined herein.

The specific-binding agents, peptides, polypeptides, proteins, biomarkers *etc.* in the present kits may be in various forms, *e.g.*, lyophilised, free in solution or immobilised on a solid phase. They may be, *e.g.*, provided in a multi-well plate or as an array or microarray, or they may be packaged separately and/or individually. The may be suitably labelled as taught herein. Said kits may be particularly suitable for performing the assay methods of the invention, such as, *e.g.*, immunoassays, ELISA assays, mass spectrometry assays, and the like.

The term "modulate" generally denotes a qualitative or quantitative alteration, change or variation specifically encompassing both increase (*e.g*., activation) or decrease (*e.g.*, inhibition), of that which is being modulated. The term encompasses any extent of such modulation.

For example, where modulation effects a determinable or measurable variable, then modulation may encompass an increase in the value of said variable by at least about 10%, *e.g.*, by at least about 20%, preferably by at least about 30%, e.g., by at least about 40%, more preferably by at least about 50%, *e.g.*, by at least about 75%, even more preferably by at least about 100%, *e.g.*, by at least about 150%, 200%, 250%, 300%, 400% or by at least about 500%, compared to a reference situation without said modulation; or modulation may encompass a decrease or reduction in the value of said variable by at least about 10%, *e.g.*, by at least about 20%, by at least about 30%, *e.g.*, by at least about 40%, by at least about 50%, *e.g.*, by at least about 60%, by at least about 70%, *e.g.*, by at least about 80%, by at least about 90%, e.g., by at least about 95%, such as by at least about 96%, 97%, 98%, 99% or even by 100%, compared to a reference situation without said modulation.

Preferably, modulation of the activity and/or level of intended target(s) (*e.g.*, LTBP2 gene or protein) may be specific or selective, *i.e.,* the activity and/or level of intended target(s) may be modulated without substantially altering the activity and/or level of random, unrelated (unintended, undesired) targets.

Reference to the "activity" of a target such as LTBP2 protein may generally encompass any one or more aspects of the biological activity of the target, such as without limitation any one or more aspects of its biochemical activity, enzymatic activity, signalling activity and/or structural activity, *e.g.*, within a cell, tissue, organ or an organism.

In the context of therapeutic or prophylactic targeting of a target, the reference to the "level" of a target such LTBP2 gene or protein may preferably encompass the quantity and/or the availability (*e.g.*, availability for performing its biological activity) of the target, *e.g*., within a cell, tissue, organ or an organism.

For example, the level of a target may be modulated by modulating the target's expression and/or modulating the expressed target. Modulation of the target's expression may be achieved or observed, e.g., at the level of heterogeneous nuclear RNA (hnRNA), precursor mRNA (pre-mRNA), mRNA or cDNA encoding the target. By means of example and not limitation, decreasing the expression of a target may be achieved by methods known in the art, such as, *e.g.*, by transfecting (*e.g.*, by electroporation, lipofection, *etc.)* or transducing (*e.g.*, using a viral vector) a cell, tissue, organ or organism with an antisense agent, such as, *e.g.*, antisense DNA or RNA oligonucleotide, a construct encoding the antisense agent, or an RNA interference agent, such as siRNA or shRNA, or a ribozyme or vectors encoding such, *etc.* By means of example and not limitation, increasing the expression of a target may be achieved by methods known in the art, such as, *e.g*., by transfecting (*e.g.*, by electroporation, lipofection, *etc.)* or transducing (*e.g.*, using a viral vector) a cell, tissue, organ or organism with a recombinant nucleic acid which encodes said target under the control of regulatory sequences effecting suitable expression level in said cell, tissue, organ or organism. By means of example and not limitation, the level of the target may be modulated via alteration of the formation of the target (such as, *e.g*., folding, or interactions leading to formation of a complex), and/or the stability (*e.g.*, the propensity of complex constituents to associate to a complex or disassociate from a complex), degradation or cellular localisation, *etc.* of the target.

The term "antisense" generally refers to a molecule designed to interfere with gene expression and capable of specifically binding to an intended target nucleic acid sequence. Antisense agents typically encompass an oligonucleotide or oligonucleotide analogue capable of specifically hybridising to the target sequence, and may typically comprise, consist essentially of or consist of a nucleic acid sequence that is complementary or substantially complementary to a sequence within genomic DNA, hnRNA, mRNA or cDNA, preferably mRNA or cDNA corresponding to the target nucleic acid. Antisense agents suitable herein may typically be capable of hybridising to their respective target at high stringency conditions, and may hybridise specifically to the target under physiological conditions.

The term "ribozyme" generally refers to a nucleic acid molecule, preferably an oligonucleotide or oligonucleotide analogue, capable of catalytically cleaving a polynucleotide. Preferably, a "ribozyme" may be capable of cleaving mRNA of a given target protein, thereby reducing translation thereof. Exemplary ribozymes contemplated herein include, without limitation, hammer head type ribozymes, ribozymes of the hairpin type, delta type ribozymes, *etc.* For teaching on ribozymes and design thereof, see, e.g., US 5,354,855, US 5,591,610, Pierce et al. 1998 (Nucleic Acids Res 26: 5093-5101), Lieber et al. 1995 (Mol Cell Biol 15: 540-551), and Benseler et al. 1993 (J Am Chem Soc 115: 8483-8484).

"RNA interference" or "RNAi" technology is routine in the art, and suitable RNAi agents intended herein may include *inter alia* short interfering nucleic acids (siNA), short interfering RNA (siRNA), double-stranded RNA (dsRNA), micro-RNA (miRNA), and short hairpin RNA (shRNA) molecules as known in the art. For teaching on RNAi molecules and design thereof, see *inter alia* Elbashir et al. 2001 (Nature 411: 494-501), Reynolds et al. 2004 (Nat Biotechnol 22: 326-30), http://rnaidesigner.invitrogen.com/rnaiexpress, Wang & Mu 2004 (Bioinformatics 20: 1818-20), Yuan et al. 2004 (Nucleic Acids Res 32(Web Server issue): W130-4), by M Sohail 2004 ("Gene Silencing by RNA Interference: Technology and Application", 1st ed., CRC, ISBN 0849321417), U Schepers 2005 ("RNA Interference in Practice: Principles, Basics, and Methods for Gene Silencing in C.elegans, Drosophila, and Mammals", 1st ed., Wiley-VCH, ISBN 3527310207), and DR Engelke & JJ Rossi 2005 ("Methods in Enzymology, Volume 392: RNA Interference", 1st ed., Academic Press, ISBN 0121827976).

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, *e.g.,* EDTA or glutathione), amino acids (such as, *e.g.*, glycine), proteins, disintegrants, binders, lubricants, wetting agents, emulsifiers, sweeteners, colorants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic compositions may be contemplated.

The present active substances (agents) may be used alone or in combination with any therapies known in the art for the disease and conditions as taught herein ("combination therapy"). Combination therapies as contemplated herein may comprise the administration of at least one active substance of the present invention and at least one other pharmaceutically or biologically active ingredient. Said present active substance(s) and said pharmaceutically or biologically active ingredient(s) may be administered in either the same or different pharmaceutical formulation(s), simultaneously or sequentially in any order.

The dosage or amount of the present active substances (agents) used, optionally in combination with one or more other active compound to be administered, depends on the individual case and is, as is customary, to be adapted to the individual circumstances to achieve an optimum effect. Thus, it depends on the nature and the severity of the disorder to be treated, and also on the sex, age, body weight, general health, diet, mode and time of administration, and individual responsiveness of the human or animal to be treated, on the route of administration, efficacy, metabolic stability and duration of action of the compounds used, on whether the therapy is acute or chronic or prophylactic, or on whether other active compounds are administered in addition to the agent(s) of the invention.

Without limitation, depending on the type and severity of the disease, a typical daily dosage might range from about 1 µg/kg to 100 mg/kg of body weight or more, depending on the factors mentioned above. For repeated administrations over several days or longer, depending on the condition, the treatment is sustained until a desired suppression of disease symptoms occurs. A preferred dosage of the active substance of the invention may be in the range from about 0.05 mg/kg to about 10 mg/kg of body weight. Thus, one or more doses of about 0.5 mg/kg, 2.0 mg/kg, 4.0 mg/kg or 10 mg/kg (or any combination thereof) may be administered to the patient. Such doses may be administered intermittently, e.g., every week or every two or three weeks.

As used herein, a phrase such as "a subject in need of treatment" includes subjects that would benefit from treatment of a given disease or condition as taught herein. Such subjects may include, without limitation, those that have been diagnosed with said condition, those prone to contract or develop said condition and/or those in whom said condition is to be prevented.

The terms "treat" or "treatment" encompass both the therapeutic treatment of an already developed disease or condition, as well as prophylactic or preventative measures, wherein the aim is to prevent or lessen the chances of incidence of an undesired affliction, such as to prevent the chances of contraction and progression of a disease or condition as taught herein. Beneficial or desired clinical results may include, without limitation, alleviation of one or more symptoms or one or more biological markers, diminishment of extent of disease, stabilised (*i.e.,* not worsening) state of disease, delay or slowing of disease progression, amelioration or palliation of the disease state, and the like. "Treatment" can also mean prolonging survival as compared to expected survival if not receiving treatment.

The term "prophylactically effective amount" refers to an amount of an active compound or pharmaceutical agent that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses for the present compounds.

The above aspects and embodiments are further supported by the following non-limiting examples.

### EXAMPLES

### Example 1: MASSterclass targeted protein quantitation for early validation of candidate markers derived from discovery

### MASSTERCLASS experimental setup

MASSterclass assays use targeted tandem mass spectrometry with stable isotope dilution as an end-stage peptide quantitation system (also called Multiple Reaction Monitoring (MRM) and Single Reaction Monitoring (SRM). The targeted peptide is specific (*i.e.,* proteotypic) for the specific protein of interest. *i.e.,* the amount of peptide measured is directly related to the amount of protein in the original sample. To reach the specificity and sensitivity needed for biomarker quantitation in complex samples, peptide fractionations precede the end-stage quantitation step.

A suitable MASSTERCLASS assay may include the following steps:
- Plasma/serum sample
- Depletion of human albumin and IgG (complexity reduction on protein level) using affinity capture with anti-albumin and anti-IgG antibodies using ProteoPrep spin columns (Sigma Aldrich)
- Spiking of known amounts of isotopically labelled peptides. This peptide has the same amino acid sequence as the proteotypic peptide of interest, typically with one isotopically labelled amino acid built in to generate a mass difference. During the entire process, the labelled peptide has identical chemical and chromatographic behaviour as the endogenous peptide, except during the end-stage quantitation step which is based on molecular mass.
- Tryptic digest. The proteins in the depleted serum/plasma sample are digested into peptides using trypsin. This enzyme cleaves proteins C-terminally from lysine and argninine, except when a proline is present C-terminally of the lysine or arginine. Before digestion, proteins are denatured by boiling, which renders the protein molecule more accessible for the trypsin activity during the 16h incubation at 37°C.
- First peptide-based fractionation: Free Flow Electrophoresis (FFE; BD Diagnostic) is a gel-free, fluid separation technique in which charged molecules moving in a continuous laminar flow are separated through an electrical field perpendicular to the flow. The electrical field causes the charged molecules to separate in the pH gradient according to their isoelectric point (pi). Only those fractions containing the monitored peptides are selected for further fractionation and LC-MS/MS analysis. Each peptide of interest elutes from the FFE chamber at a specific fraction number, which is determined during protein assay development using the synthetic peptide homologue. Specific fractions or fraction pools (multiplexing) proceed to the next level of fractionation.
- Second peptide-based fractionation: Phenyl HPLC (XBridge Phenyl; Waters) separates peptides according to hydrophobicity and aromatic nature of amino acids present in the peptide sequence. Orthogonality with the back-end C18 separation is achieved by operating the column at an increased pH value (pH 10). As demonstrated by Gilar et al. 2005, J Sep Sci 28(14): 1694-1703), pH is by far the most drastic parameter to alter peptide selectivity in RP-HPLC. Each peptide of interest elutes from the Phenyl column at a specific retention time, which is determined during protein assay development using the synthetic peptide homologue. The use of an external control system, in which a mixture of 9 standard peptides is separated upfront a batch of sample separations, allows adjusting the fraction collection in order to correct for retention time shifts. The extent of fractionation is dependent on the concentration of the protein in the sample and the complexity of that sample.
- LC-MS/MS based quantitation, including further separation on reversed phase (C18) nanoLC (PepMap C18; Dionex) and MS/MS: tandem mass spectrometry using MRM (4000 QTRAP; ABI)/SRM (Vantage TSQ; Thermo Scientific) mode. The LC column is connected to an electrospray needle connected to the source head of the mass spectrometer. As material elutes from the column, molecules are ionized and enter the mass spectrometer in the gas phase. The peptide that is monitored is specifically selected to pass the first quadrupole (Q1), based on its mass to charge ratio (m/z). The selected peptide is then fragmented in a second quadrupole (Q2) which is used as a collision cell. The resulting fragments then enter the third quadrupole (Q3). Depending on the instrument settings (determined during the assay development phase) only a specific peptide fragment or specific peptide fragments (or so called transitions) are selected for detection.
- The combination of the m/z of the monitored peptide and the m/z of the monitored fragment of this peptide is called a transition. This process can be performed for multiple transitions during one experiment. Both the endogenous peptide (analyte) and its corresponding isotopically labelled synthetic peptide (internal standard) elute at the same retention time, and are measured in the same LC-MS/MS experiment.
- The MASSterclass readout is defined by the ratio between the area under the peak specific for the analyte and the area under the peak specific for the synthetic isotopically labelled analogue (internal standard). MASSterclass readouts are directly related to the original concentration of the protein in the sample. MASSterclass readouts can therefore be compared between different samples and groups of samples.

A typical MASSTERCLASS protocol followed in the present study is given here below:
- 25µL of plasma is subjected to a depletion of human albumin and IgG (ProteoPrep spin columns; Sigma Aldrich) according to the manufacturer's protocol, except that 20mM NH₄HCO₃ was used as the binding/equilibration buffer.
- The depleted sample (225µL) is denatured for 15min at 95°C and immediately cooled on ice
- 500 fmol of the isotopically labelled peptide (custom made 'Heavy AQUA' peptide; Thermo Scientific) is spiked in the sample
- 20µg trypsin is added to the sample and digestion is allowed for 16h at 37°C
- The digested sample was first diluted 1/8 in solvent A (0.1% formic acid) and then 1/20 in the same solvent containing 250 amol/µL of all isotopically labelled peptides (custom made 'Heavy AQUA' peptide; Thermo Scientific) of interest.
- 20µL of the final dilution was separated using reverse-phase NanoLC with on-line MS/MS in MRM/SRM mode:
   - Column: PepMap C18, 75µm I.D. x 25cm L, 100 Å pore diameter, 5µm particle size
   - Solvent A: 0.1% formic acid
   - Solvent B: 80% acetonitrile, 0.1% formic acid
   - Gradient: 30 min; 2%-55% Solvent B
   - MS/MS in MRM mode: method contains the transitions for the analyte as well as for the synthetic, labelled peptide.
   - The used transitions were experimentally determined and selected during protein assay development
   - Each of the transitions of interest was measured for a period starting 3 minutes before and ending 3 minutes after the determined retention time of the peptide of interest, making sure that each peak had at least 15 datapoints.
- The raw data was analysed and quantified using the LCQuan software (Thermo Scientific): the area under the analyte (= the LTBP2 peptide) peak and under the internal standard (the labelled, synthetic LTBP2 peptide) peak at the same C18 retention time was determined by automatic peak detection. These were cross-checked manually.
- The MASSterclass readout was defined by the ratio of the analyte peak area and the internal standard peak area

### MASSTERCLASS output

The measured ratios are differential quantitations of peptides. In other words a ratio is the normalised concentration of a peptide. The concentration of a peptide is proportional to the ratio measured in the mass spectrometer.

### Example 2: Screening of acute dyspnea samples for LTBP2

In this example the clinical utility of LTBP2 measurement for the evaluation of dyspneic patients was assessed.

The 299 clinical samples used in this study are part of the BASEL V cohort, a prospective study on consecutive patients presenting themselves to the ED of the university Hospital of BASEL with dyspnea as the most prominent symptom (part of this cohort is described in Potocki et al., Journal of internal Medicine 2010 Jan;267(1):119-29). The gold standard for the diagnosis of acute heart failure was based interpretation of two independent cardiologists of all medical records pertaining to the patient including 90 day follow up data and BNP levels. Based on this, 56% (n=168) of patients were adjudicated to have an acute heart failure event, others were classified as dyspnea non-heart failure. A wide range of clinical and marker variables was collected (for summary see Table 1) including patient demographics, medical history, chronic medication, renal function parameters, echo parameters, established cardiac and inflammatory marker levels. Glomerular filtration rate was calculated using the Modification of Diet in Renal Disease (MDRD) formula (Stevens et al., New England Journal of Medicine 2006; 354:2473-83). Patients were followed up for at least 1 year post admission to the hospital and all-cause-mortality was recorded.

LTBP2 and Cystatin C levels were measured using MASSterclass™ assays as described in example 1. BNP, NT-proBNP and CRP levels were measured using commercially available immunoassays as described in Potocki et al (2010).

The diagnostic accuracy of a specific protein was determined by measuring the area under the Receiver-Operating-Characteristics (ROC) curves (AUC) as in Sullivan Pepe M (The statistical evaluation of medical tests for classification and prediction. 1993 Oxford University Press New York). The estimated and confidence intervals for AUCs were also computed using a non-parametric approach, namely bootstrapping (Efron B, Tibshirani RJ. Nonparametric confidence intervals. An introduction to the bootstrap. Monographs on statistics and applied probability. 1993; 57:75-90 Chapman & Hall New York).

Associations of LTBP2, Cystatin C, BNP, NT-proBNP and CRP levels with all available clinical parameters were computed using univariate statistical tests. Spearman's ranked test was used to compute correlation coefficients and Wilcoxon rank sum test for assessing whether two independent samples of observation originate from the same population.

**Table1: Summary of patient characteristics included in the study.**

| **Characteristic** | | **all patients (n=299)** |
|---|---|---|
| age (yr) | | 77 |
| **gender (%male)** | | 52 |
| BMI | | 26 |
| **History (%)** | | |
| | hypertension | 68 |
| | heart failure | 24 |
| | CAD | 28 |
| | diabetes | 18 |
| | COPD | 34 |
| | chronic kidney disease | 28 |
| **physical/ECG** | | |
| | heart rate | 93 ± 23 |
| | systolic bp | 138 ± 26 |
| | diastolic bp | 83 ± 16 |
| | LVEF | 24 (20-28) |
| **lab s** | | |
| | creatinin (umol/L) | 85 (66-120) |
| | eGFR (mL/min/1.73m2) | 67 (44-89) |
| | BNP (pq/mL) | 350 (90-1120) |
| | Nt-proBNP (pg/mL) | 1656 (314-6105) |
| **diagnosis (%)** | | |
| | ADHF | 56% |
| | Pneumonia | 10% |
| | Pulmonary embolism | 3% |
| | COPD/Asthma | 16% |
| | hyperventilation | 3% |
| | other | 12% |
| **outcome** | | |
| | survival at 1 yr | 73% |

### Example 3: LTBP2 associates with kidney function parameters

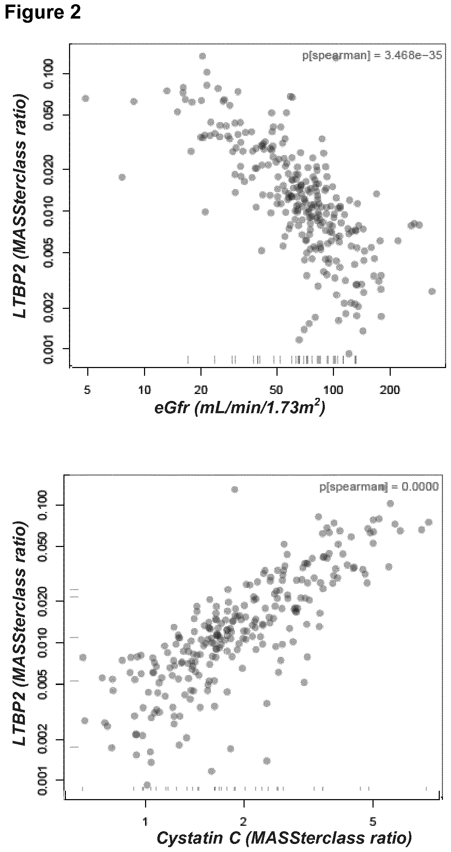

Screening acute dyspnea patients (example 2) for LTBP2 levels showed a clear association of LTBP2 level with all available clinical parameters related to kidney function as indicated by the low p-values for Spearman rank correlation with estimated glomerular filtration rate (eGfr), creatinin levels and blood urea nitrogen (BUN) levels and the low Wilcoxon p-values for presence/absence of history of kidney failure (summarized in Table 2). Figure 2 illustrates the correlation of LTBP2 with eGfr, indicating LTBP2 is a good indicator of glomerular filtration. The correlation of LTBP2 with filtration is further corroborated by correlation with Cystatin C, a known good marker for Gfr. Cystatin C was also measured in these samples using MASSterclass technology. The correlation of LTBP2 with Cystatin C and eGfr remains valid after correcting for presence of acute decompensated heart failure (Table 2).

**Table 2: Summary statistics on univariate associations for Cystatin C and LTBP2. Values mentioned for Spearman ranked test are correlation coefficients between 2 continuous variables, Wilcoxon test returns p-values which show significance of association between continuous marker levels and 2 discrete populations.**

| | **Cystatin C** | **LTBP2** | **Population** | **statistic** |
|---|---|---|---|---|
| creatinin | 0 | 6.66134E-16 | AHF | spearman |
| creatinin | 0 | 4.99463E-11 | dyspnea non AHF | spearman |
| glomerular filtration rate (gfr) | 4.94586E-23 | 1.67122E-17 | AHF | spearman |
| glomerular filtration rate (gfr) | 1.43419E-19 | 3.87316E-14 | dyspnea non AHF | spearman |
| blood urea nitrogen (BUN) | 0 | 8.88178E-16 | AHF | spearman |
| blood urea nitrogen (BUN) | 0 | 1.67022E-12 | dyspnea non AHF | spearman |
| History of kidney failure | 7.47895E-16 | 1.35514E-10 | AHF | wilcoxon |
| History of kidney failure | 4.59187E-06 | 1.82318E-05 | dyspnea non AHF | wilcoxon |

### Example 4: LTBP2 as a marker of renal dysfunction

Estimated glomerular filtration rate is a good indicator of how well the kidneys are functioning. Patients with eGfr below 60 for a minimum of 3 months are considered to have chronic kidney disease (CKD). The acute dyspnea population under study has 107 patients with reduced eGfr vs 192 patients with more normal glomerular filtration rates.

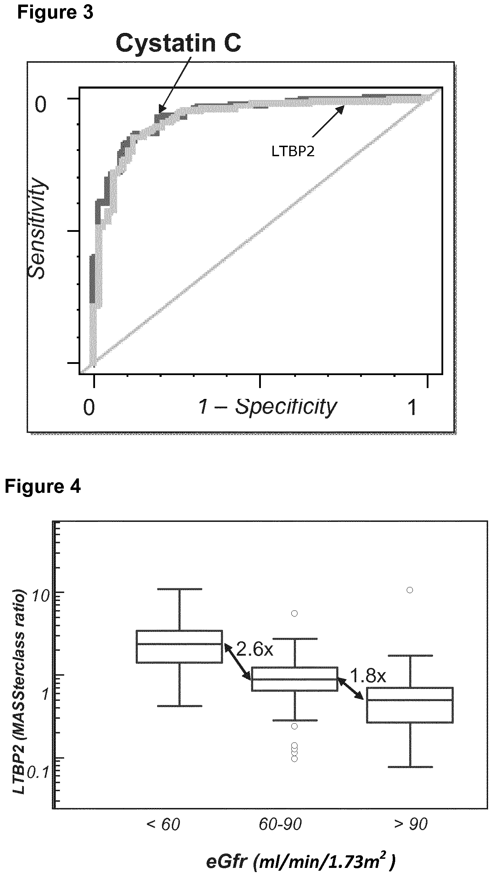

Receiver-operating characteristics (ROC) analysis demonstrated LTBP2 to be highly sensitive and specific for diagnosing kidney dysfunction in this population of dyspneic patients, as indicated by an overall median AUC of 0.9 with 95% Cl 0.85-0.93 (Figure 3). This diagnostic performance is equivalent to Cystatin C, the best available biomarker for chronic kidney disease.

Figure 4 illustrates relative levels of LTBP2 as measured by MASSterclass in patients with reduced (<60), intermediate (60-90) and normal (>90) estimated glomerular filtration rates. Median LTBP2 levels among patients with patients with reduced eGfr were 4,7 fold higher than patients with normal eGfr (>90). LTBP2 levels are also elevated in patients with slightly reduced kidney function (eGfr between 60 and 90).

### Example 5: LTBP2 as a marker for acute changes in renal function

The correlation of LTBP2 with Cystatin C and eGfr remains valid after correcting for presence of acute decompensated heart failure. The correlation in AHF patients hints to the fact that LTBP2 can detect acute changes in eGfr due to the acute decompensation of the heart (i.e., reduced cardiac output).

After restoring cardiac output and renal function by therapeutic intervention LTBP2 levels also return to baseline levels.

### Example 6: LTBP2 as predictive marker for mortality

In the cohort of acute dyspnea patients under study patients were followed up for at least one year post admission. At 1 year post admission, 82/299 subjects (27%) had died (all-cause mortality). The relation of LTBP2 and other clinical and marker variables to mortality was studied using different methods. Receiver-operator characteristic analysis with death at 1 year as the reference standard were performed and median area under the curve was calculated. Distributions of marker levels in "alive" and "death" patients were compared using the Wilcoxon rank-sum test. Kaplan Meier curves compared mortality rates across the follow-up period after presentation in groups divided as a function of LTBP2 levels.

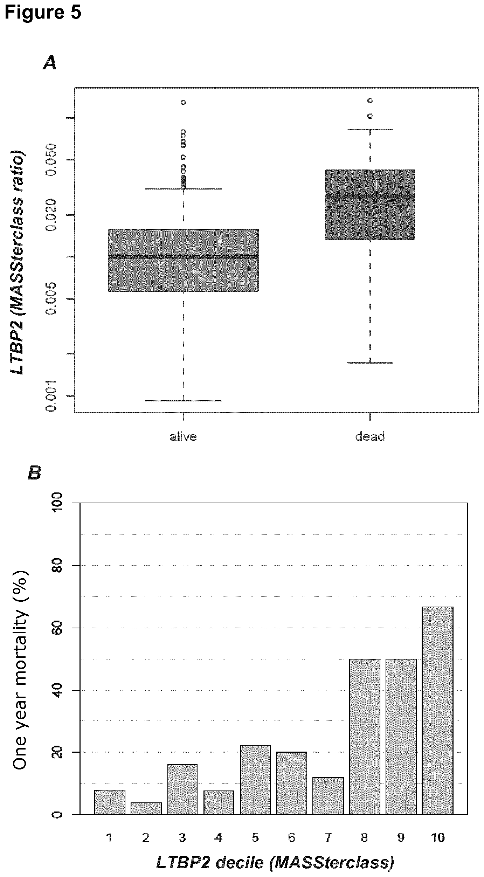

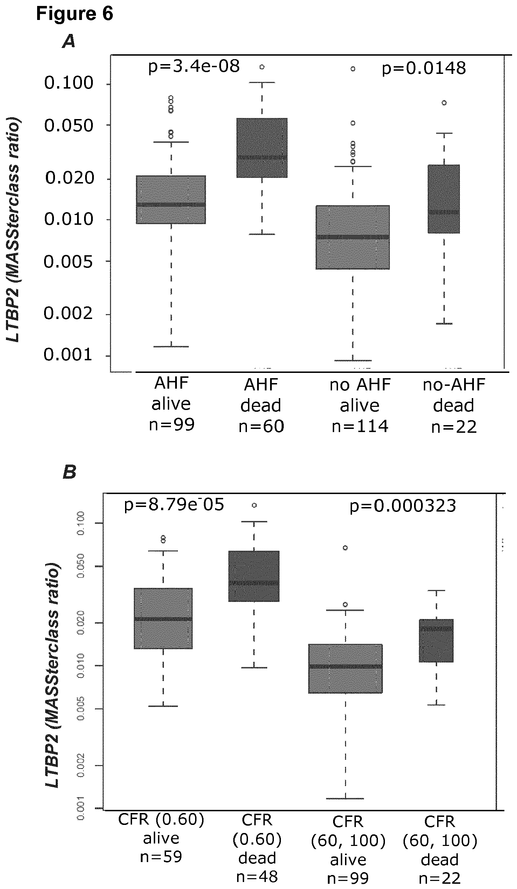

Concentrations of LTBP2 at presentation in patients with acute dyspnea were significantly higher among patients who died by 1 year (n=82; 27%) compared with patients who were alive (p = 2e⁻¹¹) (Figure 5A). This pattern of higher LTBP2 concentrations in decedents remained when subjects were considered as a function of the presence (p=3.5e⁻⁰⁸) or absence of acute heart failure (p=0.01) (Figure 6A) and when the population was divided based on renal function (eGfr < 60; p=8.8e-05 vs eGfr >60; p=0.0003) (Figure 6B). This illustrates LTBP2 has the potential to predict bad outcome in a general dyspneic population as well as in an acute heart failure population and a chronic kidney disease population.

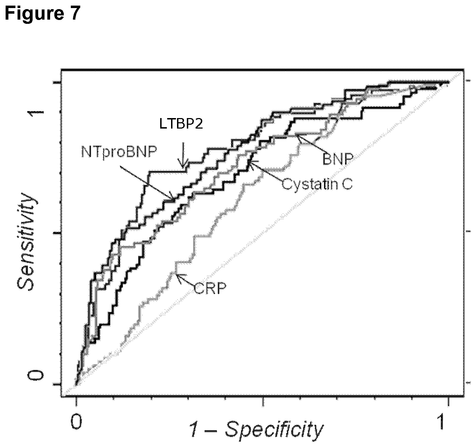

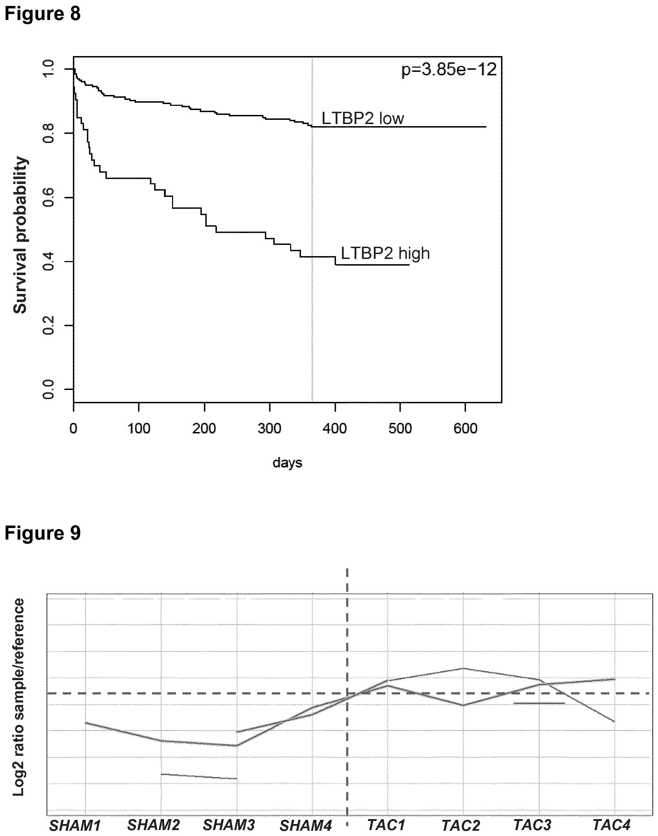

In addition decile analysis of LTBP2 concentrations examined as a function of mortality rates at 1 year revealed that there was a graded increase in mortality with rising concentrations of the marker (Figure 5B). ROC analysis performed for predicting death at 1 year in all acute dyspnea patients demonstrated an AUC of 0.77 for LTBP2 (95% Cl: 0.7-0.84), similar to NT-proBNP (AUC = 0.77, but higher than BNP, Cystatin C and CRP protein markers (Figure 7). Kaplan Meier analysis shows rates of death rise rapidly from admission up to 1 year for those patients with LTBP2 above the cut-off point at maximum accuracy (Figure 8).

Multivariable Cox proportional hazard analysis using forward stepping were performed to identify the independent predictors of death at 1 year for this patient cohort. Variables were retained if their univariable p-value was < 0.05 and entered into a multivariable model; hazard ratios (HR) were generated and only those variables with significant p values were retained in the final multivariable model. In this multivariate analysis LTBP2 levels above the cut off for maximum accuracy is a strong independent predictor of death at 1 yr in all dyspneic patients (HR = 3.76; p<0.0001). Table 3 summarizes the selected univariable and multivariable predictors of 1 year mortality. Of note the final selected multivariable model contains LTBP2 combined with measures for renal function (eGfr and urea), bmi and potassium indicating LTBP2 can show complementarity over this variables for predicting survival.

**Table 3: Selected univariable and multivariable predictors of 1-year mortality in dyspneic patients (HR = hazard ratio; Cl = confidence interval)**

| | ***Univariable*** | | | ***Multivariable*** | | |
|---|---|---|---|---|---|---|
| **Variable** | **HR** | **95% Cl** | **p-value** | **HR** | **95% Cl** | **p**-**value** |
| age (yr) | 2.49 | 1.73-3.58 | < 0.0001 | | | |
| admission weight | 0.73 | 0.55-0.96 | 0.0281 | | | |
| weight at discharge | 0.52 | 0.35-0.79 | 0.00169 | | | |
| admission bmi | 0.66 | 0.49-0.887 | 0.00569 | 0.55 | 0.4-0.768 | 0.000388 |
| admission systolic blood pressure | 0.60 | 0.44-0.83 | 0.00167 | | | |
| admission diastolic blood pressure | 0.72 | 0.54-0.98 | 0.0336 | | | |
| admission oxygen saturation | 0.87 | 0.75-1.01 | 0.0629 | | | |
| admission oxygen therapy | 1.51 | 1.31-1.75 | < 0.0001 | | | |
| admission respiratory rate | 1.43 | 1.16-1.76 | 0.000832 | | | |
| myoglobin ( | 1.83 | 1.44-2.32 | < 0.0001 | | | |
| Potassium (mmol/L) | 1.19 | 1.12-1.26 | < 0.0001 | 1.11 | 1.03-1.18 | 0.00374 |
| eGfr (mL/min/1.73m2) | 0.57 | 0.46-0.71 | < 0.0001 | 1.72 | 1.02-2.9 | 0.0404 |
| Blood urea nitrogen (mmol/L) | 2.51 | 1.91-3.29 | < 0.0001 | 2.13 | 1.19-3.84 | 0.0112 |
| uric acid | 1.87 | 1.36-2.57 | 0.000121 | | | |
| albumin | 0.60 | 0.49-0.73 | < 0.0001 | | | |
| hemoglobin (9/L) | 0.71 | 0.59-0.84 | < 0.0001 | | | |
| hematocrit | 0.66 | 0.53-0.84 | 0.000487 | | | |
| LVEF (%) | 0.64 | 0.42-0.99 | 0.0467 | | | |
| Troponin T (ug/L) | 1.81 | 1.48-2.21 | < 0.0001 | | | |
| Cystatin C (MASSterclass ratio) | 2.20 | 1.65-2.92 | < 0.0001 | | | |
| LTBP2 (MASSterclass ratio) | 3.46 | 2.47-4.85 | < 0.0001 | 3.76 | 2.13-6.64 | < 0.0001 |
| BNP (pg/mL | 2.97 | 2.03-4.34 | < 0.0001 | | | |
| NTproBNP (pg/mL) | 4.20 | 2.78-6.35 | < 0.0001 | | | |
| CRP (mg/L) | 1.64 | 1.18-2.26 | 0.00279 | | | |

### Example 7: LTBP2 as a marker for cardiac left ventricular hypertrophy

Thoracic aortic constriction (TAC) is a well established model of left ventricular hypertrophy and eventually heart failure. Four animals per group (SHAM control and TAC group) undergo operation and a clip is put on the ascending aorta of the TAC group. Rats are monitored by echocardiography and myocardial performance is calculated. After 4 weeks animals are sacrificed and blood is collected by cardiac puncture. The heart is subsequently perfused with PBS and the ventricles (left and right) are dissected and snap frozen in liquid nitrogen. Protein lysates from left ventricles were prepared using standard protocols and MASStermind technology combined with subsequent statistical analysis (see below) was used to determine differences in protein profiles of hypertrophied left ventricles (the TAC group) versus SHAM controls.

In summary, samples were prepared for MASStermind analysis according to the standard N-terminal COFRADIC procedures. Samples and controls were differentially labelled by trypsin mediated incorporation of ¹⁸O/¹⁶O at the C-terminus of every tryptic peptide. After N-terminal peptide sorting, NanoLC separations followed by direct spotting onto MALDI targets were performed. MALDI-TOF/TOF instrumentation was used to generate MS spectra. The MS spectra were analyzed using in-house developed bioinformatics tools, such as tools for peak recognition and de-isotoping, ratio determination between analyte and reference, clustering, inter-sample alignment and extensive sample quality control. Once all the samples were aligned and quality controlled, statistical analysis was initiated. To select for differential features (or peptides) that discriminate two populations, two different statistical measures were applied: one-rule classifier and Significance Analysis of Microarrays (SAM) analysis. Conceptually the simplest machine learning technique to find differential features is a one-rule classifier. In this method a simple rule of the form "If ratio < X then class = A else class = B" is generated for each feature. The performance of this rule on the data is determined by leave-one-out cross-validation. Features that show low error rates in this analysis are prime biomarker candidates. SAM (Tusher et al. 2001, PNAS 98: 5116-5121) is a method to select the most differential features, while controlling the False Discovery Rate (FDR). The main advantage of this method over the one-rule classifier is that it will still allow to pick up useful trends when the difference in ratios between both classes start to diminish and random noise from the experiment starts to obscure the actual levels of the candidate markers. SAM calculates the relative difference in the ratio of features between two classes of samples. To estimate the significance of this score, a null distribution is estimated by permuting the class assignments of all samples and re-scoring. This gives us a confident estimation of the false discovery rat (FDR), that is the percentage of proteins or gene products that were identified by chance. To optimally account for missing values and intensity of the MS signal, the complete SAM analysis was run on different subsets of the data, using different cut-offs for these values. All results were compiled in a final report.

As illustrated in figure 9, LTBP2 and a close family member (LTBP4) show increased levels in hypertrophied left ventricles compared to SHAM controls. This difference is statistically significant as indicated by SAM analysis scores (<0.1% false discovery rate).

### Example 8: LTBP2 expression in a mouse model of kidney fibrosis.

An independent link of LTBP2 levels to kidney dysfunction was retrieved from Gene Expression Omnibus (GEO), a public repository of gene expression profiles (http://www.ncbi.nlm.nih.gov/geo/). Searching the database for LTBP2 expression profiles highlighted the highly increased levels of LTBP2 transcript in kidneys of Gli2 knockout mice. Glis2 is a transcription factor belonging to the family of Kruppel-like zinc finger proteins with a critical role in maintaining normal renal function (for review: Kang et al., 2010, Histology and Histopathology).

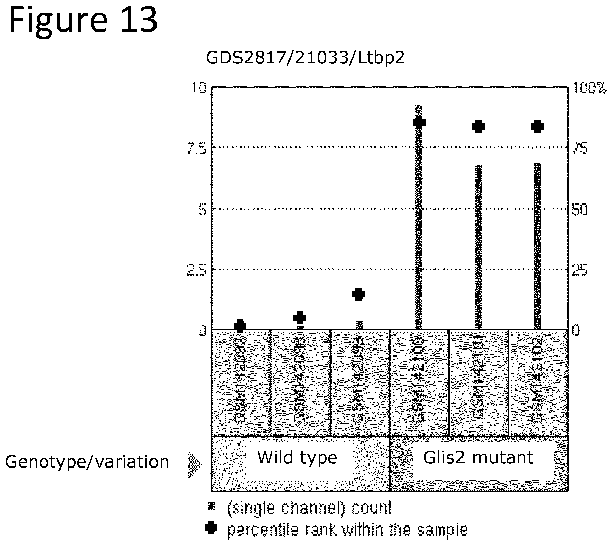

The Glis2 knockout mice model was described as a good model for nephronophthisis (a kidney disease characterized by extensive fibrosis which progressively leads to end-stage renal disease) and is considered the best mouse model for chronic kidney disease development (Attanasio et al., 2007, Nature Genetics). At birth Glis2 knockout mice do not uncover any obvious developmental abnormalities in the kidneys. However from 4 weeks to 6 months, kidneys from the knockout mice substantially decrease in size and weight. By 6 months there is significant interstitial fibrosis with deposition of collagen throughout the kidney. Gene expression profiling using the GE Healthcare Codelink system on kidneys at 4 weeks of age from wild type and knockout animals indeed showed a 40 fold increase in expression for LTBP2 in the wild type animals (Figure 13). This increase in expression occurs at a timepoint before obvious histological differences in the kidneys are observed and can thus be seen as having predictive value.

### Example 9: LTBP2 expression in human kidney biopsies.

The distribution of LTBP2 protein in human kidneys was subsequently analyzed by immunohistochemistry using a polyconal antibody as described in Hyytiainen et al., (1998, Journal of Biological Chemistry). Human kidney biopsies were obtained from human patients diagnosed with kidney tumors. Large parts of the kidney of these patients are still histologically normal but as these patients are on average 40-50 years old they have weak atherosclerosis leading to a thickening of the kidney intima.

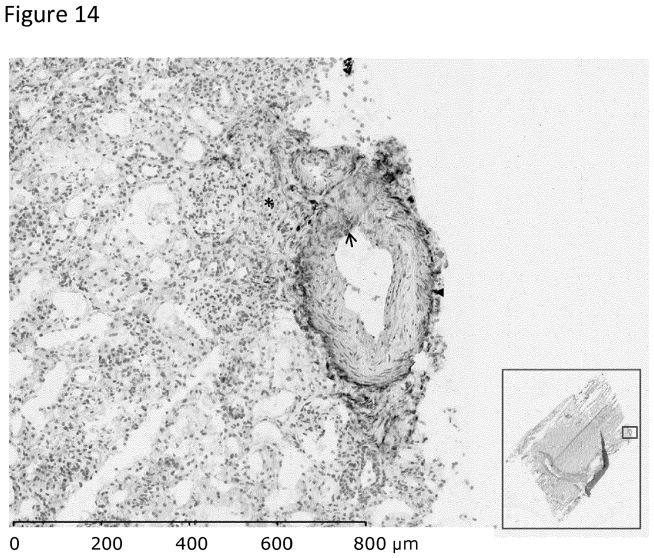

The polyclonal antibody highlights expression of LTBP2 in the perivasculature (Figure 14, arrow), both in endothelial and smooth muscle cells. Expression is also observed in the intima (Figure 14, *) which is pathologically thickened in these patients due to onset of atherosclerosis. The intima is an extra-cellular matrix structure with deposition of collagen structures. The thickening of the intima is considered a pro-fibrotic process which can lead to progressive kidney fibrosis under pathological conditions.

## Claims

1. A method for predicting or prognosticating mortality in a subject having dyspnea and/or acute heart failure, comprising measuring the quantity of LTBP2 in a sample from said subject, wherein said sample is blood, serum, plasma or urine.

2. The method according to claim 1 comprising the steps of:
(i) measuring the quantity of LTBP2 in a sample from the subject;
(ii) comparing the quantity of LTBP2 measured in (i) with a reference value of the quantity of LTBP2, said reference value representing a known prediction or prognosis of mortality;
(iii) finding a deviation or no deviation of the quantity of LTBP2 measured in (i) from the reference value; and
(iv) attributing said finding of deviation or no deviation to a particular prediction or prognosis of mortality in the subject.

3. The method according to claim 2 wherein an elevated quantity of LTBP2 in a sample from the subject compared to a reference value representing the prediction prognosis of a given mortality within a predetermined time interval indicates that the subject has a comparably greater risk of deceasing within said time interval.

4. The method according to any one of claims 1 to 3, wherein the method further comprises measuring the presence or absence and/or quantity of one or more other biomarkers useful for predicting or prognosticating mortality in the subject having dyspnea and/or acute heart failure in the sample from the subject.

5. The method according to claim 4 comprising:
(i) measuring the quantity of LTBP2 and the presence or absence and/or quantity of said one or more other biomarkers in the sample from the subject;
(ii) using the measurements of (i) to establish a subject profile of the quantity of LTBP2 and the presence or absence and/or quantity of said one or more other biomarkers ;
(iii) comparing said subject profile of (ii) to a reference profile of the quantity of LTBP2 and the presence or absence and/or quantity of said one or more other biomarkers, said reference profile representing a known prediction or prognosis of mortality;
(iv) finding a deviation or no deviation of the subject profile of (ii) from the reference profile;
(v) attributing said finding of deviation or no deviation to a particular prediction or prognosis of mortality in the subject.

6. The method according to claims 4 or 5, wherein said other biomarker is chosen from the group consisting of creatinine, Cystatin C, neutrophil gelatinase-associated lipocalin (NGAL), beta-trace protein, kidney injury molecule 1 (KIM-1), interleukin-18 (IL-18), B-type natriuretic peptide (BNP), pro-B-type natriuretic peptide (proBNP), amino terminal pro-B-type natriuretic peptide (NTproBNP) and C-reactive peptide.

7. The method according to any one of claims 1 to 6, wherein the quantity of LTBP2 and/or the presence or absence and/or quantity of the one or more other biomarkers is measured using, respectively, a binding agent capable of specifically binding to LTBP2, and a binding agent capable of specifically binding to said one or more other biomarkers.

8. The method according to any one of claims 1 to 7, wherein the quantity of LTBP2 and/or the presence or absence and/or quantity of the one or more other biomarkers is measured using an immunoassay technology, or using a mass spectrometry analysis method or using a chromatography method, or using a combination of said methods.

## Patentansprüche

1. Verfahren zur Vorhersage oder Prognose der Mortalität bei einem Patienten, der an Dyspnoe und/oder akuter Herzinsuffizienz leidet, umfassend die Messung der LTBP2-Menge in einer Probe dieses Patienten, wobei es sich bei dieser Probe um Blut, Serum, Plasma oder Urin handelt.

2. Verfahren gemäß Anspruch 1, weiterhin enthaltend die Schritte:
(i) Messung der LTBP2-Menge in einer Probe des Patienten;
(ii) Vergleich der in (i) gemessenen LTBP2-Menge bezogen auf einen Referenzwert der LTBP2-Menge, wobei dieser Referenzwert eine bekannte Mortalitätsvorhersage oder-Prognose repräsentiert;
(iii) Finden einer Abweichung oder Nicht-Abweichung der in (i) gemessenen LTBP2-Menge vom Referenzwert; und
(iv) Zuordnen der genannten aufgefundenen Abweichung oder Nicht-Abweichung zu einer speziellen Mortalitätsvorhersage oder-Prognose bei dem Patienten.

3. Verfahren gemäß Anspruch 2, wobei eine erhöhte LTBP2-Menge in einer Probe des Patienten, verglichen mit dem Referenzwert, der die vorhergesagte Prognose einer gegebenen Mortalität innerhalb eines vorher festgelegten Zeitintervalls repräsentiert, darauf hinweist, dass der Patient ein vergleichsweise höheres Risiko hat, innerhalb dieses Zeitintervalls zu versterben.

4. Verfahren gemäß einem der Ansprüche 1 bis 3, wobei das Verfahren weiterhin die Messung des Vorhandenseins oder des Fehlens und/oder der Menge eines oder mehrerer anderer Biomarker(s) in der Probe des Patienten, der an Dyspnoe und/oder akuter Herzinsuffizienz leidet, die zur Mortalitätsvorhersage oder-Prognose nützlich sind, umfasst.

5. Verfahren gemäß Anspruch 4 umfassend:
(i) Messung der LTBP2-Menge und des Vorhandenseins oder des Fehlens und/oder der Menge eines oder mehrerer anderer Biomarker(s) in der Probe des Patienten;
(ii) Verwendung der Messungen aus (i), um ein Patientenprofil der LTBP2-Menge und des Vorhandenseins oder Fehlens und/oder der Menge des genannten einen oder der mehreren anderen Biomarker zu erstellen;
(iii) Vergleich dieses Patientenprofils aus (ii) mit einem Referenzprofil der LTBP2-Menge und des Vorhandenseins oder des Fehlens und/oder der Menge dieses einen oder der mehreren anderer Biomarker, wobei dieses Referenzprofil eine bekannte Mortalitätsvorhersage oder-Prognose repräsentiert;
(iv) Finden einer Abweichung oder Nicht-Abweichung des Patientenprofils aus (ii) von diesem Referenzprofil;
(v) Zuordnen der genannten aufgefundenen Abweichung oder Nicht-Abweichung zu einer speziellen Mortalitätsvorhersage oder-Prognose bei dem Patienten.

6. Verfahren gemäß Anspruch 4 oder 5, wobei dieser andere Biomarker aus der Gruppe ausgewählt wird, die aus: Kreatinin, Cystatin C, Neutrophilen Gelatinaseassoziiertem Lipocalin (NGAL), Beta-Trace-Protein, Nierenschädiguns-Molekül 1 (KIM-I), Interleukin-18 (1L-18), B-Typ natriuretischem Peptid (BNP), pro-B-Typ natriuretischem Peptid (proBNP) und amino-terminalem pro-B-Typ natriuretischem Peptid (NTproBNP) und C-reaktives Peptid besteht.

7. Verfahren gemäß einem der Ansprüche 1 bis 6, wobei die Menge des LTBP2 und/oder des Vorhandenseins oder Fehlens und/oder der Menge des genannten einen oder der mehreren anderen Biomarker unter Verwendung jeweils eines Bindemittels, das in der Lage ist, sich spezifisch an LTBP2 zu binden, sowie eines Bindemittels, das in der Lage ist, sich spezifisch an den genannten einen oder die mehreren anderen Biomarker zu binden, gemessen wird.

8. Verfahren gemäß einem der Ansprüche 1 bis 7, wobei die Menge des LTBP2 und/oder des Vorhandenseins oder Fehlens und/oder der Menge des genannten einen oder der mehreren anderen Biomarker unter Verwendung einer Immunoassay-Technologie oder unter Verwendung einer Massenspektrometer-Analysemethode, oder einem Chromatographieverfahren oder unter Verwendung einer Kombination der genannten Verfahren gemessen wird.

## Revendications

1. Méthode pour prédire ou pronostiquer la mortalité chez un sujet souffrant de dyspnée et/ou d'insuffisance cardiaque aiguë, comprenant la mesure de la quantité de LTBP2 dans un échantillon obtenu dudit sujet, dans laquelle ledit échantillon est du sang, du sérum, du plasma ou de l'urine.

2. Méthode selon la revendication 1, comprenant les étapes :
(i) mesurer la quantité de LTBP2 dans un échantillon obtenu du sujet;
(ii) comparer la quantité de LTBP2 mesurée en (i) avec une valeur de référence de la quantité de LTBP2, ladite valeur de référence représentant une prédiction ou un pronostic connu de mortalité;
(iii) constater un écart ou une absence d'écart entre la quantité de LTBP2 mesurée en (i) et la valeur de référence; et
(iv) attribuer ladite constatation d'écart ou d'absence d'écart à une prédiction ou un pronostic particulier de mortalité chez le sujet.

3. Méthode selon la revendication 2, dans laquelle une quantité élevée de LTBP2 dans un échantillon obtenu du sujet comparée à une valeur de référence représentant le pronostic de prédiction d'une mortalité donnée dans un intervalle de temps prédéterminé indique que le sujet présente un risque comparativement plus grand de décès dans ledit intervalle de temps.

4. Méthode selon l'une quelconque des revendications 1 à 3, dans laquelle la méthode comprend en outre la mesure de la présence ou de l'absence et/ou de la quantité d'un ou plusieurs autres biomarqueurs utiles pour prédire ou pronostiquer la mortalité chez le sujet souffrant de dyspnée et/ou d'insuffisance cardiaque aiguë dans l'échantillon obtenu du sujet.

5. Méthode selon la revendication 4, comprenant:
(i) mesurer la quantité de LTBP2 et la présence ou l'absence et/ou la quantité dudit un ou plusieurs autres biomarqueurs dans l'échantillon obtenu du sujet;
(ii) utiliser les mesures de (i) pour établir un profil du sujet de la quantité de LTBP2 et la présence ou l'absence et/ou la quantité dudit un ou plusieurs autres biomarqueurs;
(iii) comparer ledit profil du sujet de (ii) à un profil de référence de la quantité de LTBP2 et la présence ou l'absence et/ou la quantité dudit un ou plusieurs autres biomarqueurs, ledit profil de référence représentant une prédiction ou un pronostic connu de mortalité;
(iv) constater un écart ou une absence d'écart du profil du sujet de (ii) par rapport au profil de référence;
(v) attribuer ladite constatation d'écart ou d'absence d'écart à une prédiction ou à un pronostic de mortalité chez le sujet.

6. Méthode selon les revendications 4 ou 5, dans laquelle ledit autre biomarqueur est choisi dans le groupe constitué de la créatinine, la cystatine C, la lipocaline associée à la gélatinase neutrophile (NGAL), la protéine bêta-trace, la molécule de lésion rénale 1 (KIM-1), l'interleukine 18 (IL-18), le peptide natriurétique de type B (BNP), le peptide natriurétique de type pro-B (proBNP), la partie amino-terminale du peptide natriurétique de type pro-B (NTproBNP) et le peptide C-réactif.

7. Méthode selon l'une quelconque des revendications 1 à 6, dans laquelle la quantité de LTBP2 et/ou la présence ou l'absence et/ou la quantité du ou des autres biomarqueurs est mesurée en utilisant, respectivement, un agent liant capable de se lier spécifiquement à LTBP2 et un agent liant capable de se lier spécifiquement audit un ou plusieurs autres biomarqueurs.

8. Méthode selon l'une quelconque des revendications 1 à 7, dans laquelle la quantité de LTBP2 et/ou la présence ou l'absence et/ou la quantité du ou des autres biomarqueurs est mesurée au moyen d'une technologie de dosage immunologique, ou en utilisant une analyse de spectrométrie de masse, ou en utilisant un procédé de chromatographie, ou en utilisant une combinaison desdites méthodes.
